# EUROPEAN PATENT SPECIFICATION

(11) **EP 1 916 312 B1**
(45) Date of publication and mention of the grant of the patent: **11.01.2012**
(21) Application number: 07254236.8
(22) Date of filing: 25.10.2007
(51) Int. Cl.: C12Q 1/68, C12N 9/12

(54) **Use of DNA polymerases as exoribonucleases**
Verwendung von DNA-Polymerasen wie Exoribonukleasen
Utilisations d'ADN polymérases comme exoribonucleases

(30) Priority: 26.10.2006 GB 0621361
(43) Date of publication of application: 30.04.2008
(73) Proprietor: Fermentas UAB, Vilnius 2028 (LT)
(72) Inventor: Lagunavicius, Arunas, Vilnius 09104 (LT); Janulaitis, Arvydas, Vilnius 08122 (LT); Kiveryte, Zivile, Vilnius 09314 (LT); Zimbaite-Ruskuliene, Vilma, Vilnius 07130 (LT); Savaneviciute, Agne, Vilnius region 13250 (LT)
(74) Representative: Daniels, Jeffrey Nicholas

(56) References cited:
- WO-A-02/50310
- WO-A-97/20948
- WO-A-99/49079
- WO-A-2006/108422
- NAKAI H ET AL: "DISSECTION OF RNA-PRIMED DNA SYNTHESIS CATALYZED BY GENE 4 PROTEIN AND DNA POLYMERASE OF BACTERIOPHAGE T-7 COUPLING OF RNA PRIMER AND DNA SYNTHESIS" JOURNAL OF BIOLOGICAL CHEMISTRY, vol. 261, no. 32, 1986, pages 15217-15224, XP002468316 ISSN: 0021-9258
- RAMADAN K ET AL: "Human DNA Polymerase lambda Possesses Terminal Deoxyribonucleotidyl Transferase Activity And Can Elongate RNA Primers: Implications for Novel Functions" JOURNAL OF MOLECULAR BIOLOGY, LONDON, GB, vol. 328, no. 1, 18 April 2003 (2003-04-18), pages 63-72, XP004457613 ISSN: 0022-2836
- POWELL M D ET AL: "Residues in the alphaH and alphaI helices of the HIV-1 reverse transcriptase thumb subdomain required for the specificity of RNase H-catalyzed removal of the polypurine tract primer." THE JOURNAL OF BIOLOGICAL CHEMISTRY 9 JUL 1999, vol. 274, no. 28, 9 July 1999 (1999-07-09), pages 19885-19893, XP002468317 ISSN: 0021-9258
- GORSHKOVA I I ET AL: "HIV-1 reverse transcriptase interaction with model RNA-DNA duplexes." ANALYTICAL BIOCHEMISTRY 15 APR 2001, vol. 291, no. 2, 15 April 2001 (2001-04-15), pages 198-206, XP002468318 ISSN: 0003-2697
- LARSSON C ET AL: "IN SITU GENOTYPING INDIVIDUAL DNA MOLECULES BY TARGET-PRIMED ROLLING-CIRCLE AMPLIFICATION OF PADLOCK PROBES" NATURE METHODS, XX, XX, vol. 1, no. 3, 18 November 2004 (2004-11-18), pages 227-232, XP009068999 ISSN: 1548-7091
- NELSON J R: "PHI29 DNA POLYMERASE-BASED METHODS FOR GENOMICS APPLICATIONS" JOURNAL OF CLINICAL LIGAND ASSAY, CLINICAL LIGAND ASSAY SOCIETY, WAYNE, MI, US, vol. 25, no. 3, October 2002 (2002-10), pages 276-279, XP009041683 ISSN: 1081-1672
- JONSTRUP SOREN PETER ET AL: "A microRNA detection system based on padlock probes and rolling circle amplification.", RNA (NEW YORK, N.Y.) SEP 2006, vol. 12, no. 9, September 2006 (2006-09), pages 1747-1752, ISSN: 1355-8382

## Description

The present invention relates to new uses of DNA polymerases, especially Phi29 DNA polymerase. The invention also relates to processes and kits for polynucleotide production using DNA polymerases.

Rolling circle amplification (RCA) as a method of polynucleotide production is well known in the art. RCA is based on the rolling replication of short single stranded, template DNA circles by a DNA polymerase. In a basic RCA method, an exogenous primer hybridised to the template DNA circle is extended by a DNA polymerase, and a complementary DNA strand is formed. The polymerase continuously progresses around the circle, creating linear repeats complementary to the template, because it is able to displace the complementary strand ahead of it. Phi29 DNA polymerase is often used in such methods in part because of its good strand displacement activity. Accordingly, unlike polymerase chain reaction (PCR) (an alternative method of DNA amplification) a heating step to denature the double stranded DNA templates is not required and RCA can be performed at one temperature.

The basic RCA method described above is known as linear RCA. However, when two exogenous primers are used RCA can also be exponential. In this case, while the first primer hybridises to the template DNA circle as with linear RCA, the second primer hybridises to the complementary DNA product. Accordingly, the produced DNA strand can also act as a template for further replication. As a result, replication of the template proceeds exponentially. This method is also known as double-primed, hyper-branched, ramification or cascade RCA.

Rolling circle amplification can be used in large-scale polynucleotide production; in the synthesis of DNA and RNA oligomers, and their analogues, for use as probes and diagnostic and/or therapeutic agents (US-B-6368802).

Rolling circle amplification is also often used in the detection of nucleic acids and other molecules in diagnostic genomics and proteomics; for example, the *in situ* detection of a specific RNA molecule in a tissue section. In these methods a circular oligonucleotide template is created using a padlock probe (Baner*, et al.,* NAR, 1998); Lizardi *et al.,* 1998). Padlock probes are initially linear oligonucleotides with two specific sections of sequence, one at either end, connected by a linker region. Each end section is complementary to a target DNA or RNA sequence. When these sections hybridise to the target sequence they become juxtaposed and can be joined by a DNA ligase to form a single stranded circle, with a double stranded section that is hybridized to the target DNA or RNA.

Both of the end sections are required to hybridise to the target in order for RCA to be permitted. Further, ligation must also occur, with mismatched hybridization significantly affecting the efficiency of the ligation reaction. Accordingly, RCA of the circularized probe is highly specific and is able to discriminate between point mutations in the target DNA or RNA.

Different variations of RCA may be used for amplification of DNA, including amplification and analysis of complete genomes (Haible *et al.,* 2003; Niel *et al.,* 2005), and also for single or multiplex detection of all macromolecules already identified in the course of genome projects: DNA, RNA, proteins and different proximity probes (Landegren *et al.,* 2004). RCA has been shown as a useful method for detection of not only isolated or preformed targets *in vitro,* but also *in situ,* for analysis of single molecules (Christian *et al.,* 2001; Larsson *et al.,* 2004).

Detection and quantity measurements of RNA molecules by RCA is routinely performed using immuno-RCA (Zhou *et al.,* 2001), by primer DNA ligation - circularization on RNA template followed by RCA (Nilsson *et al.,* 2001; Christian *et al.,* 2001) and by using padlock probes (PP) in cDNA analysis (Baner *et al.,* 2003; Baner *et al.,* 2005).

The disadvantage of these methods is that they all require DNA primers to initiate RCA. Since RCA is a very sensitive reaction and padlock assays are often used in the multiparallel analysis of multiple different targets, the addition of exogenous RCA primers adds a further process step and may decrease reaction specificity due to possible non-specific hybridization of them to other padlock probes and targets, or due to possible contamination. In addition, it is more expensive for the customers to have to purchase additional primers.

Detection of RNA molecules by RCA, using RNA target as primer for RCA, was previously described for miRNA (Jonstrup, *et al.,* 2006). However the RNA target - padlock probe duplexes described in this publication were perfectly matched at the 3' end of miRNA and RCA was performed without degradation of RNA. The disadvantage of this method is that target sequence must be located only near the end of RNA template, so it is impossible to test targets distant from the 3' end of RNA. In certain cases this limitation is absolute, e.g. in eukaryotic mRNA molecules which have long polyA sequences at the 3' end it is impossible to test particular individual transcript.

WO 2006/108422 A discloses methods for production of oligonucleotides. WO 99/49079 A discloses rolling circle replication of padlock probes. Nakai et al., (1986) discloses a dissection of RNA-primed DNA synthesis catalyzed by gene 4 protein and DNA polymerase of bacteriophate T7. Ramadan et al., (2003) discloses that human DNA polymerase λ possesses terminal deoxyribonucleotidyl transferase activity and can elongate RNA primers. Powell et al., (1999) discloses that residues in the aH and al helices of the HIV-1 reverse transcriptase thumb subdomain are required for the specificity of RNase H-catalyzed removal of the polypurine tract primer. Gorshkova et al., (2001) discloses HIV-1 reverse transcriptase interaction with model RNA-DNA duplexes.

The present invention aims to overcome the disadvantages associated with the methods of the prior art. In particular, the present invention aims to provide a method of RCA using padlock probes which can be used with template RNA containing target sequence in any position of RNA molecule, but which does not require additional DNA primers.

Accordingly, the present invention provides the use of a DNA polymerase enzyme as a single stranded exoribonuclease.

In one aspect, the present invention provides a process for hydrolysing a single stranded RNA sequence comprising contacting the RNA sequence with a DNA polymerase enzyme having exoribonuclease activity.

The present inventors have discovered that DNA polymerases possess intrinsic RNase activity against single stranded RNA. Further, they have discovered that this property can be used to both augment and extend the various applications of DNA polymerases. In particular, as explained further below, the property can be advantageously applied to a method of amplifying an RNA template, allowing for the direct use of an RNA template as a primer for padlock probe RCA without the intermediary steps of DNA primer use.

As indicated above, the DNA polymerases used in the present invention are those with intrinsic RNase activity. In particular, the part of these polymerases responsible for the 3' to 5' exonuclease activity of the polymerase forms a fold containing a Ribonuclease H-like motif. More particularly, the polymerase is one in which the 3'-5' exonuclease coding sequence or the 3'-5 exonuclease domain has a fold containing a Ribonuclease H-like structure- motif - i.e. the polymerase is one in which the part of the polymerase sequence responsible for the 3' to 5' exonuclease activity forms a fold containing a Ribonuclease H-like structure- motif.

As is known by a person skilled in the art an RNase H fold motif is defined by a particular spatial arrangement of protein alpha and beta structures. Depending on the function performed by the domain displaying the RNase H fold motif, proteins are classified into five superfamilies. One of these superfamilies is the Ribonuclease H superfamily, which comprises nucleases.

The family members of the Ribonuclease H-like superfamily are shown in Table 1.

**Table 1 - Superfamily: Ribonuclease H-like**

| | | | | | | | | |
|---|---|---|---|---|---|---|---|---|
| 1. **Class** | | | | | | | | |
| | Alpha and beta proteins (a/b) | | | | | | | |
| | | 1. **Fold** | | | | | | |
| | | | Ribonuclease H-like motif | | | | | |
| | | | | 1. **Superfamily** | | | | |
| | | | | | Ribonuclease H-like | | | |
| | | | | | | 1. **Family** | | |
| | | | | | | | 1. | 3'-5' exonuclease domain of archaeal and phage |
| | | | | | | | | DNA polymerases (2) |
| | | | | | | | | [d1clqa1] |
| | | | | | | | | [d1noya] |
| | | | | | | | | [d1t7pa1] |
| | | | | | | | | [d1tgoa1] |
| | | | | | | | | |
| | | | | | | | 2. | Exonuclease domain of DNA polymerase (1) |
| | | | | | | | | [d1kfsa1] |
| | | | | | | | | [d1qtma1] |
| | | | | | | | | [d1xw1] |
| | | | | | | | | |
| | | | | | | | 3. | Retroviral integrase (2) |
| | | | | | | | | [d1b9da] |
| | | | | | | | | [d1cxqa] |
| | | | | | | | | |
| | | | | | | | 4. | Ribonuclease H (2) |
| | | | | | | | | [d1ril ] |
| | | | | | | | | [d1vrta1] |
| | | | | | | | | [d2rn2 ] |
| | | | | | | | | |
| | | | | | | | 5. | RuvC resolvase (1) |
| | | | | | | | | [d1hjra ] |
| | | | | | | | | |
| | | | | | | | 6. | Transposase inhibitor (Tn5 transposase) (1) |
| | | | | | | | | [d1b7ea ] |
| | | | | | | | | |
| | | | | | | | 7. | homol.tod1b9da (1) |
| | | | | | | | | [c1c6va ] |
| | | | | | | | | |
| | | | | | | | 8. | homol. to d2rn2 (1) |
| | | | | | | | | [c1j12b ] |
| | | | | | | | | |
| | | | | | | | 9. | mu transposase, core domain (1) |
| | | | | | | | | [d1bco 2] |

A 3'-5' exonuclease coding sequence can be identified at the level of amino acid sequence similarity comparisons. In some proteins the 3'-5' exonuclease coding sequence forms a separate protein domain which is identifiable from their 3D structure. In other cases proteins do not have spatially identifiable domains, and different enzymatic activities (such as exonuclease or polymerase activities) are realised within the same domain, as in the case of phi29 DNA polymerase.

The comparison to identify polymerases having a 3'-5' exonuclease coding sequence or a 3'-5 exonuclease domain with a fold containing a Ribonuclease H-like structure-active site motif can be performed using any suitable fold recognition software. Such software includes web-based programs such as that provided by the 3D-PSSM server, which is a web-based fold recognition server run by Imperial College of Science, Technology and Medicine (London, UK). The DNA polymerase can be assessed as having a ribonuclease H-like structure-active site motif on the basis of different confidence predictions. Preferably, a 95% confidence prediction is used.

The polymerase enzyme may be selected from the group consisting of archaeal and phage DNA polymerases.

Preferably the polymerase enzyme is selected from T4 DNA polymerase (B class polymerase), T7 DNA polymerase (A class polymerase) or Phi29 DNA polymerase (B class polymerase), although Phi29 DNA polymerase, from the *Bacillus subtilis* phage phi29 (Φ29), is most preferred. The amino acid sequences for these DNA polymerases are provided as SEQ ID No: 25, 26 and 27 respectively.

As described above, phi 29 DNA polymerase is a monodomain protein, which possesses polymerase activity (encoded by the C terminal part of the protein) and 3' to 5' exonuclease (encoded by the N terminal part of the protein). Analysis of the 240 N-terminal amino acids of phi 29 DNA polymerase indicates that this part of the protein is similar to T7 polymerase 3' -5' exonuclease domain with 95% confidence prediction.

In particular, the DNA polymerase of the present invention is capable of acting as a single stranded RNA exoribonuclease and hydrolysing a strand of RNA.

One aspect of the present invention comprises the use of a DNA polymerase as a single stranded exoribonuclease for production of a ribonucleotide. In particular the ribonucleotide is a 5'-ribomonophosphate.

A further aspect of the present invention comprises the use of a DNA polymerase as a single stranded RNA exoribonuclease for producing a RNA-DNA fusion molecule.

In a preferred aspect of the invention the use of a DNA polymerase enzyme as a single stranded RNA exoribonuclease is in a method of polynucleotide production. In particular, this can be the synthesis or amplification of DNA from an RNA template or RNA target. The polynucleotides may be produced from any suitable dNTP and include both naturally occurring and synthetic nucleotides, and modified bases. Suitable nucleotides are those that can be incorporated into the growing DNA strand by the DNA polymerase.

In this and other aspects of the present invention the DNA polymerase may be used as a combined polymerase and single stranded exoribonuclease. Specifically, in one embodiment the polymerase enzymatically converts the target RNA molecule into a primer for DNA polymerization. In particular, this can be a primer for DNA polymerization via RCA. This use reduces the risk of sample contamination, leads to simpler handling and reduces the cost of each RCA reaction. Increased specificity is also achieved as the absence of non-specific hybridization of the exogenous DNA primers, which are not added.

This aspect has particular advantages within the use of a DNA polymerase enzyme as an exoribonuclease in a method for *in situ* detection of RNA. Such a use can be applied to the *in situ* detection of an RNA locus or species, a single nucleotide polymorphism (SNP), a splice variant, or a deletion or insertion into the RNA.

In particular, this aspect of the present invention provides a process for polynucleotide production from a target region within a single stranded RNA sequence comprising: a) forming a padlock probe-target region hybrid; b) adding a DNA polymerase enzyme capable of acting as a single stranded RNA exoribonuclease; c) allowing the enzyme to hydrolyse the RNA sequence; and d) allowing the enzyme to act as a polymerase to produce the polynucleotide in the presence of dNTPs, wherein the process is conducted in the absence of an exogenous primer. In a specific embodiment of this aspect the DNA polymerase is Phi29 DNA polymerase.

A padlock probe precursor is a linear oligonucleotide with two end segments, connected by a linker region. Both end segments comprise sequence which is complementary to the target sequence. When both ends become hybridised to the target sequence and ligated to produce circle structure the oligonucleotide is then known as a padlock probe.

Generally step a) is carried out by hybridising and ligating a padlock probe or a padlock probe precursor to the target region.

The nature of the RNA sequence is not particularly limited. The sequence may be at least 25 nucleotides, preferably at least 50 nucleotides, and most preferably at least 100 nucleotides.

In one embodiment of the present invention, the RNA sequence is an eukaryotic mRNA. In a preferred aspect it is an mRNA with a poly (A) tail.

The target region within the RNA sequence may be located anywhere along the length of the sequence. In a preferred aspect the target region does not include the terminal ribonucleotide or the RNA sequence.

In particular, the above process is conducted in the absence of an exogenous primer. The term "exogenous primer" is meant to refer to additional primers that have previously been used in the RCA methods of the prior art to hybridise to the padlock probe and initiate DNA polymerisation by the DNA polymerase. In the present invention, the use of such a primer is unnecessary since the RNA template is digested by the polymerase to form a primer. Therefore, the process is conducted in the absence of a further primer (additional to the padlock probe or padlock probe precursor which are themselves sometimes referred to in the art as primers, which would hybridise to the padlock probe sequence.

However, the method of the present invention may require further primers if the RCA is to be exponential. Where exponential amplification of polynucleotide from a target region within an RNA sequence is required the process of the present invention requires a primer complementary to the produced polynucleotide so that the produced polynucleotide itself can become a template for further polynucleotide production.

In the process of the present invention the enzyme may be contacted with the RNA sequence in the presence of a divalent metal ion. Suitable divalent metal ions include Mg²⁺, Mn²⁺ and Co²⁺.

Further, the process may additionally comprise contacting the polymerase with the RNA molecule in the presence of RNAse. Long stretches of single stranded RNA are known to form intra-molecular RNA-RNA structures which can inhibit the exoribonuclease activity of the DNA polymerase. This can be prevented with the use of an RNAse which can hydrolyse regions of double stranded RNA. Further, the addition of RNAses during hybridisation or before hybridisation stimulates the formation of RNA-DNA hybrids. Preferably the RNAse is selected from RNAse III or RNAse VI.

In addition, the padlock probe or padlock probe precursor can be hybridised to the target region in the presence of Rec A protein. It has been indicated that Rec A protein can promote the formation of RNA-DNA hybrids (Kirkpatrick *et al.,* 1992). The present inventors have discovered that the use of Rec A in RCA experiments promotes the formation of RNA-padlock probe precursor and RNA-padlock probe hybrids.

In a further aspect of the present invention a process is provided for detecting an RNA molecule comprising the RNA sequence which process comprises the above process and a step wherein a labelled probe is contacted with the produced polynucleotide to detect the RNA molecule.

In particular, the RNA molecule may be detected *in situ,* which allows the detection of the RNA in its cellular environment. Alternatively, the RNA molecule may be detected *in vitro* from total RNA, and, in particular, total mRNA. Total RNA refers to the entire population of RNA molecules which may be separated or extracted from a particular source; for example, from a particular cell type.

In a further aspect the present invention provides a kit for polynucleotide production from a target region-within a single stranded RNA sequence comprising a padlock probe or a padlock probe precursor, and a DNA polymerase capable of acting as a single stranded RNA exoribonuclease, which does not contain a further or additional primer other than the probe or probe precursor. In an embodiment of this aspect the DNA polymerase is Phi29 DNA polymerase. The kit preferably comprises dNTPs (to be used in the polynucleotide production) and may further comprise a DNA ligase to ligate the padlock probe to the target RNA.

In particular, the kit can be used for detection of RNA targets *in situ.* RNA targets may be an RNA locus or species, a single nucleotide polymorphism (SNP), a splice variant, or a deletion or insertion in the RNA. In these cases it is preferable for the kit to comprise further a labelled hybridisation probe. The kit may also comprise an RNase and/or a Rec A protein.

In one aspect of the invention it is envisaged that the kit described above will be used for polynucleotide production.

The kit of the present invention may be provided with instructions. In particular, the kit may comprise instructions for using the polymerase as an exoribonuclease. Further, the kit may comprise instructions for using the kit in the absence of an exogenous primer. The instructions may be provided on the kit packaging, printed on a leaflet accompanying the kit, or encoded in a data storage medium accompanying or remote from the kit.

The invention will now be described by way of example only, with reference to the following experiments and specific embodiments, and the accompanying drawings, in which:
FIGURE 1 shows a graph of the RNase activity of Phi29 DNA polymerase on [3H] poly A RNA substrate.
FIGURE 2 shows two graphs of the RNase activity of Phi29 DNA polymerase in two different buffer systems in the presence of different cofactors.
FIGURE 3 shows the gel photographs of the results of the studies described in Example 3.
FIGURE 4 shows the gel photographs of the results of the studies described in Example 4
FIGURE 5 shows the gel photographs of the results of the studies described in Example 5.
FIGURE 6 shows the gel photographs of the results of the studies described in Example 6.
FIGURE 7 shows the gel photographs of the results of the studies described in Example 7.
FIGURE 8 shows the gel photographs of the results of the studies described in Example 8.
FIGURE 9 shows the gel photographs of the results of the studies described in Example 9.
FIGURE 10 shows the gel photographs of the results of the studies described in Example 10.
FIGURE 11 shows the gel photographs of the results of the studies described in Example 11.
FIGURE 12 shows the gel photographs of the results of the studies described in Example 12.
FIGURE 13 shows the gel photographs of the results of the studies described in Example 13.
FIGURE 14 shows the gel photographs of the results of the studies described in Example 14.
FIGURE 15 shows the gel photographs of the results of the studies described in Example 15.
FIGURE 16 shows the gel photographs of the results of the studies described in Example 16.
FIGURE 17 shows the gel photographs of the results of the studies described in Example 17.
FIGURE 18 shows the gel photographs of the results of the studies described in Example 18.
FIGURE 19 shows the gel photographs of the results of the studies described in Example 19.
FIGURE 20 shows the gel photographs of the results of the studies described in Example 20.
FIGURE 21 shows the photographs of the results of the studies described in Example 21.
FIGURE 22 shows the photographs of the results of the studies described in Example 22.
FIGURE 23 shows the photographs of the results of the studies described in Example 23.

### 1. RNase activity of polymerases

### 1.1.Bioinformatics

The 3D-PSSM server is a fold recognition server run by Imperial College of Science, Technology and Medicine (London, UK). It provides a web based method for protein fold recognition using 1D and 3D sequence profiles coupled with secondary structure and solvation potential information.

Pfam is operated by the Wellcome Trust Sanger Institute (UK), and is an online collection of protein families and domains. Pfam contains multiple protein alignments and profile-HMMs of these families.

Bioinformatics analysis of N-terminal part of Phi29 DNA polymerase sequence (which comprises sequence encoding 3'-5' exonuclease function of the enzyme) on 3D-PSSM server, http://www.sbg.bio.ic.ac.uk/~3dpssm/index2.html, with a 95% confidence prediction revealed that Phi29 polymerase sequence encoding 3'-5' exonuclease function has fold containing Ribonuclease H- like motif, hence the enzyme belongs to Ribonuclease H- like Superfamily, similarly like 3'-5' exonucleolytic domains of T4 or T7 DNA polymerases.

The identified fold shared by the enzymes is adapted to cleave either DNA or RNA, so Ribonuclease H-like superfamily includes ribonucleases and deoxyribonucleases of the following Pfam members: 3_5_exonuc; CAF1; DDE; DNA_pol_B_exo; Exonuc_X-T; Mu_transposase; Phage_Lacto_M3; Piwi; RnaseH; RNase_HII; RuvC; rve; Transposase_11 http://www.sanger.ac.uk/cgi-bin/Pfam/getacc?PF00075.

### 1.2. Experimental ascertainment of Phi29 polymerase RNase activity

To verify if Phi29 polymerase can hydrolyze RNA, RNA cleavage experiments were carried out using [³H] poly(A) RNA as substrate. RNA degradation activity was determined by formation of acid soluble products. The experimental detail is provided as "Example 1" below.

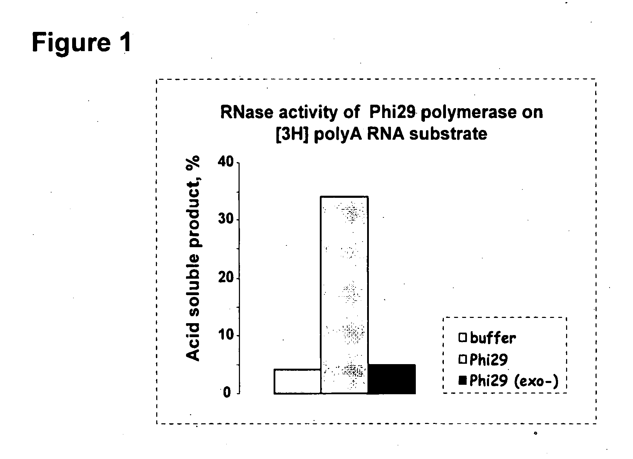

We have found that Phi29 polymerase exhibits distinctive RNase activity: incubation with Phi29 polymerase increased the amount of acid soluble product by 30% when compared with the control sample with no enzyme added (Figure 1). To ensure that these findings are not the result of experimental contamination and that the same amount of polymerase always has the same corresponding amount of RNase activity, RNA cleavage experiments were carried out with equal amounts of different Phi29 DNA polymerase lots and versions (exo+ and exo-). Our data show that mutant of Phi29 exonucleolytic center was catalytically inactive, thereby supporting the presumption that RNase activity of Phi29 could be related with said center. Moreover, RNase activity was not influenced by the presence of dNTPs in the reaction mixture (at final concentration 1mM), what allowed to presume that this activity could be exploited in some widely used applications of Phi29 DNA polymerase, namely, RCA directly from RNA targets.

### 1.3. Cofactors of Phi29 polymerase RNase activity

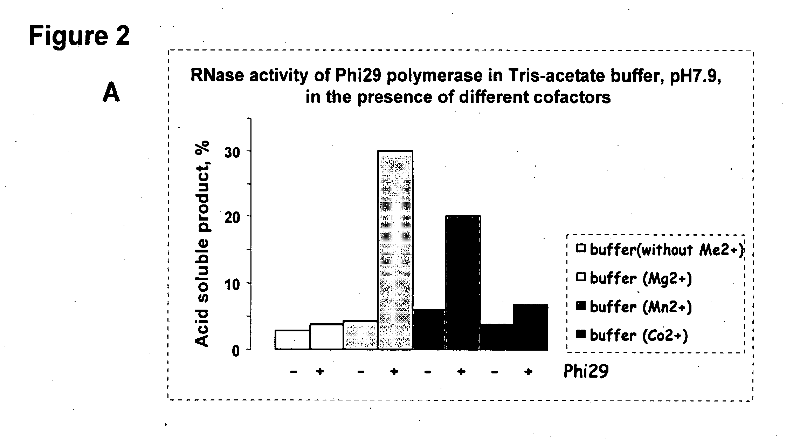

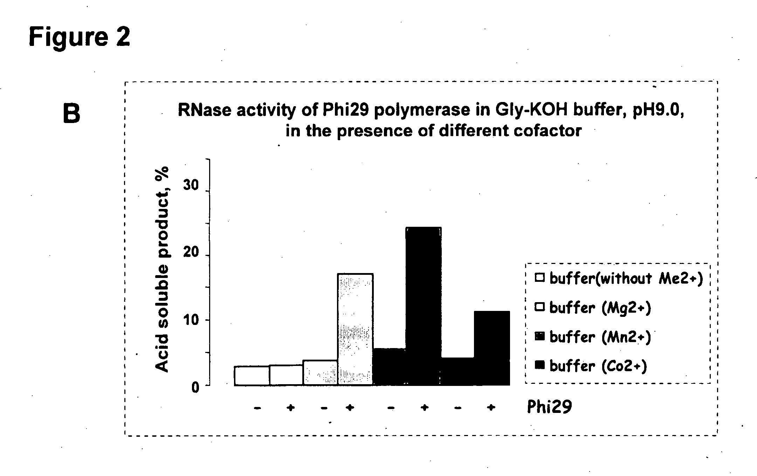

Detected RNase activity of Phi29 polymerase was partially characterized. The experimental detail is provided as "Example 2" below. It was shown that the enzyme is catalytically active in various buffer systems and needs bivalent metal ions as cofactors, such as Mg2+, Mn2+, Co2+ (Fig. 2). Depending on the reaction buffer composition cofactor suitability for RNA hydrolysis may differ. For example, in Tris - acetate buffer, pH7.9, Phi29 DNA polymerase better degrades RNA in the presence of Mg2+ (Fig. 2, part A), while in Gly-KOH buffer, pH9.0, the enzyme prefers Mn2+ ions (Fig. 2, part B).

### 1.4. Type of RNA hydrolysis by Phi29 polymerase

Possibility to employ novel Phi29 polymerase RNase activity depends on the type of this activity (exonucleolytic or endonucleolytic), hence, experiments were performed to determine type of RNA hydrolysis. The experimental detail is provided as "Example 3" below.

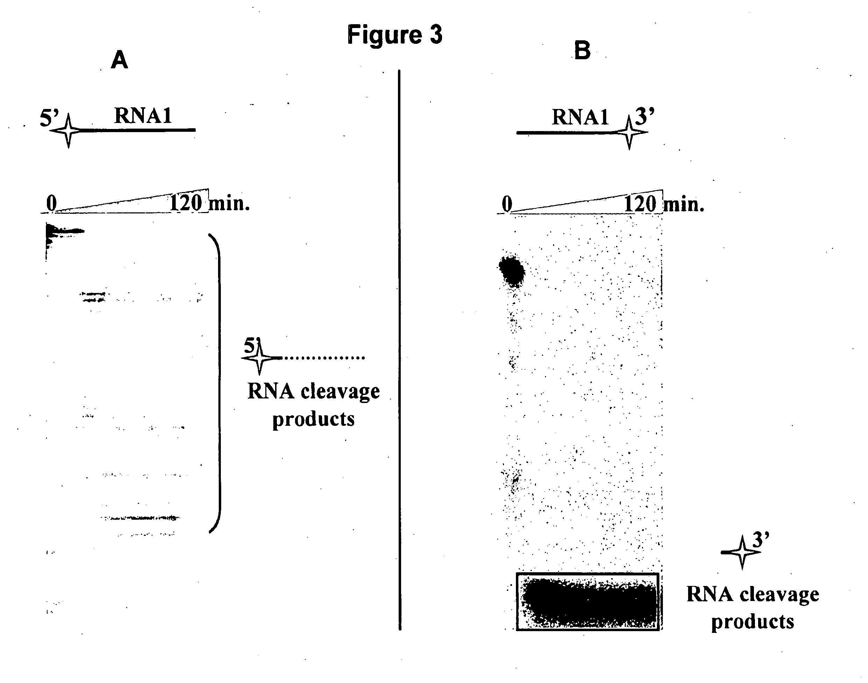

Hydrolysis of synthetic 48 nt RNA oligonucleotide RNA1, labeled at either 5'- or 3'-end was performed with Phi29 polymerase and accumulation of hydrolysis products was monitored (Fig. 3, parts A and B, respectively). Data show that in case of 5'- end labeled substrate degradation of RNA molecule results in shortened 5'-labeled products (Fig.3, part A), while degradation of 3'- end labeled RNA substrate results in the accumulation of small labeled product (Fig. 3, part B). This allows to presume that Phi29 polymerase processively degrades RNA by gradually releasing nucleotides from the 3'-end.

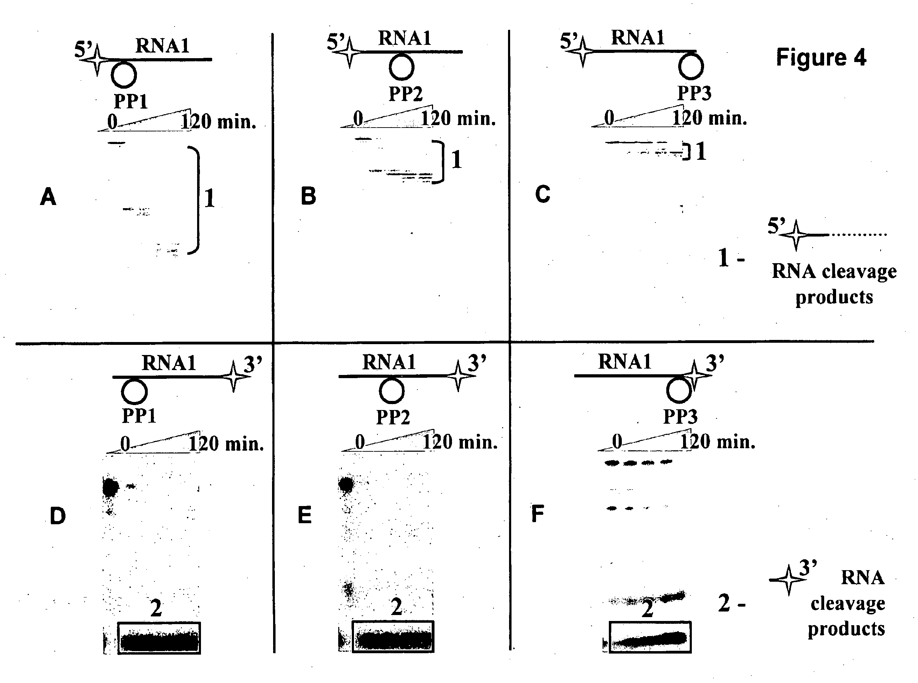

Further Phi29 action on RNA-DNA hybrids was tested. The experimental detail is provided as "Example 4" below. Analogous kinetics experiments were done with several hybrids, composed from RNA oligonucleotide RNA1, labeled at 5'- or 3'- end (Fig. 4, parts A-C and D-F, respectively) and preformed circular single stranded DNA molecules - padlock probes PP1, PP2 ir PP3, complementary to 5'- end, center pert or 3'- end of RNA1 molecule (Fig. 4, A and D, B and E, C and F parts, respectively). Circular DNA molecules for hybrids were chosen in order to prevent DNA degradation by 3'-5' exodeoxyribonuclease activity of Phi29 DNA polymerase.

Kinetics experiments on DNA-RNA confirmed that, like in case of RNA hydrolysis (Fig. 3), Phi29 polymerase recessively hydrolyses RNA in 3'-5' direction: in case of 5'- end labeled RNA - PP substrates degradation of RNA molecule was evidenced by shortening 5'-labelled products (Fig.4, parts A-C), while in case of 3'- end labeled RNA - PP substrates accumulation of small labeled product was observed (Fig.4, parts D-F). Interestingly, that RNA hydrolysis products differed depending on the location of padlock probe hybridization with RNA molecule: the closer hybridization locus was to the labeled 5'- end, the shorter RNA hydrolysis products were formed (Fig. 4, parts A-C). This indicates that Phi29 polymerase stops RNA degradation at RNA-DNA hybridization locus.

### 1.5. Final products of Phi29 polymerases 3'-5' exoribonucleolytic degradation - reason for enzyme stop

The experimental detail for this section is provided as "Example 5" below.

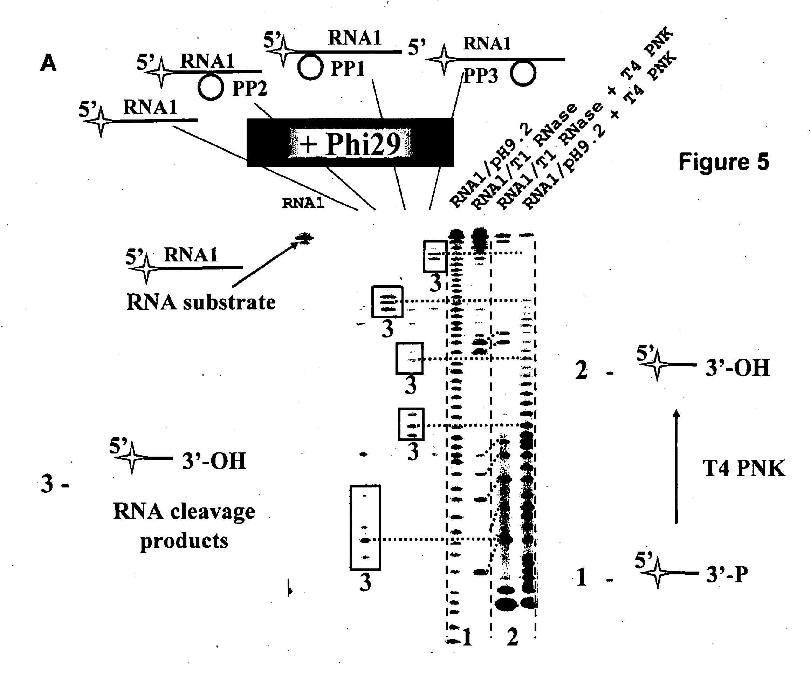

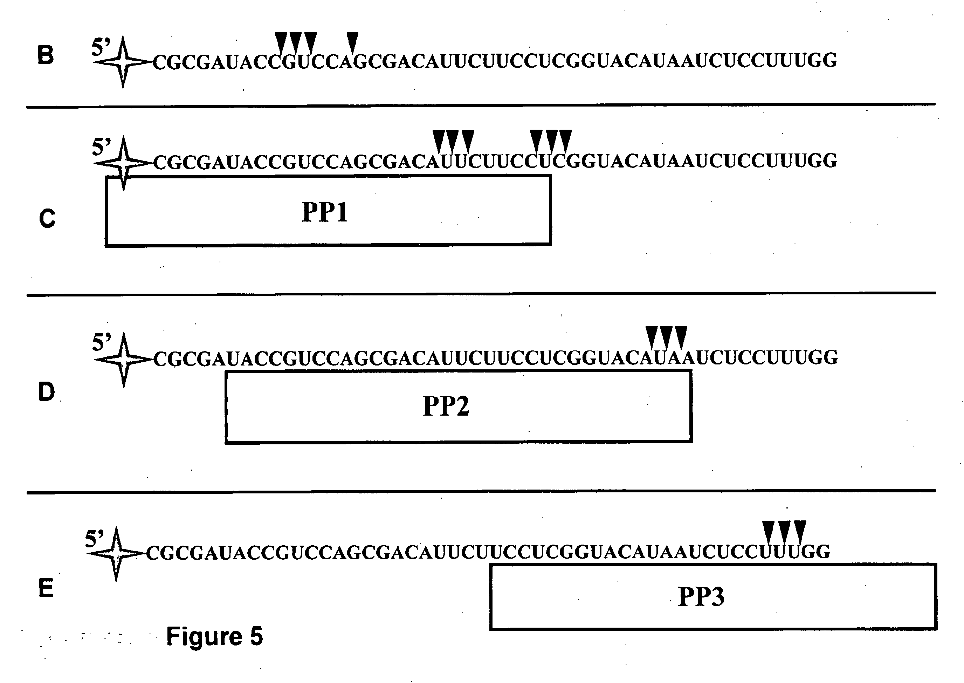

Final products of RNA1 oligonucleotide hydrolysis (both in free state and in RNA-DNA duplexes) were identified by RNA alkaline hydrolysis using RNA1 oligonucleotide markers produced by RNazesT1 cleavage (cleavage specificity - G) (Fig. 5, part A). Electrophoretic mobility of final products generated by Phi29 polymerase RNase activity was compared with that of the relevant marker fragments having phosphates or dephosphorylated at 3' ends (dephosphorylation with PNK). It was demonstrated that Phi29 polymerase hydrolyses RNA forming 3'-OH products (Fig.5, part A). Also it was shown that the reason for stop of 3'-5' exoribonukleolytic RNA degradation in RNA- DNA hybrids was the formation of double stranded structure: Phi29 polymerase degraded free RNA1 to final products of 9-14 nt length (Fig. 5, part B), while in hybrids with padlock probes polymerase paused either at the beginning of double stranded structure (Fig. 5, parts C and D) or slightly inside hybridization area (Fig. 5, parts C and E). Such RNA degradation in RNA- DNA hybrid area is evidently much slower process (see Fig. 4, parts C, F and Fig. 3, parts A, B, respectively) and possibly could be explained by base pairing disruption during the "breathing" of double stranded structure. Summarized data on enzyme kinetics and final products identification indicate that Phi29 polymerase is processive 3'-5' exoribonuclease, which efficiently hydrolyses single stranded RNA, while inefficiently and only partially hydrolyses RNA involved into double stranded RNA-DNA structures: RNA hydrolysis in hybrids was observed only in the 3' terminal part of the duplex.

These data are in good concordance with results obtained in labeled transcripts hydrolysis experiments, when measuring the fraction of radioactive acid soluble product. Contrary to the polyA RNA hydrolysis, in the same conditions Phi29 polymerase did not show notable RNase activity on transcripts most probably due to the formation of intramolecular double stranded RNA-RNA structures at the 3'-end of the transcript.

### 2. Enzymatic conversion of RNA target into primer for RCA reaction, using preformed padlock-probes

### 2.1. Target RNA conversion into primer for RCA by Phi29 polymerase

The experimental detail for this section is provided as "Example 6" and "Example 7" below.

The fact that Phi29 polymerase exhibits RNase activity even in the presence of dNTPs allowed to presume that enzyme 3'-5' exoribonucleolytic activity could find the use in target RNA conversion into a primer for RCA: the enzyme RNase activity should degrade RNA up to the double stranded region of probe hybridization making RNA into primer from which in the presence of dNTPs it should begin synthesis of DNA complementary to the padlock probe.

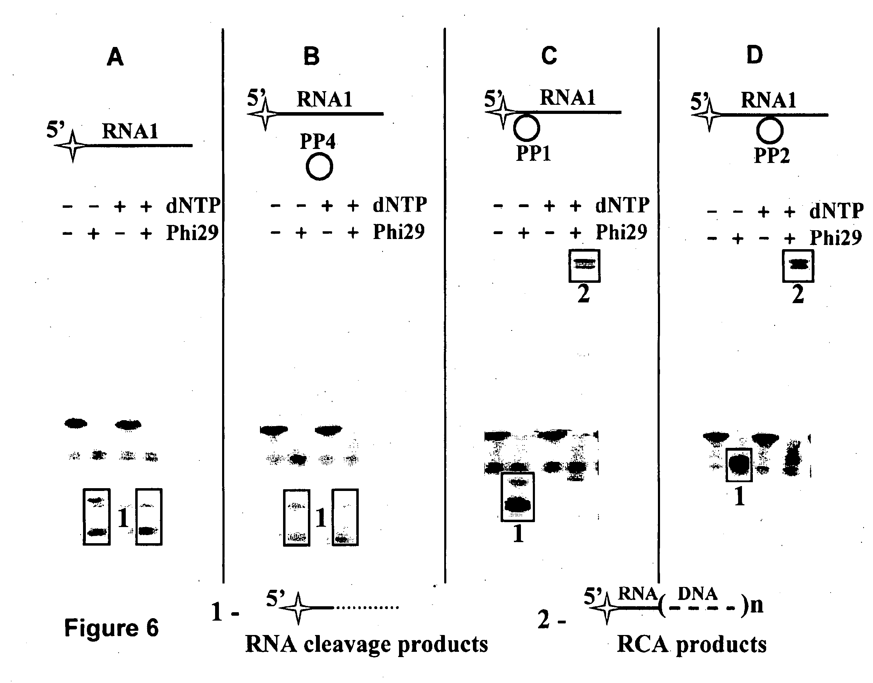

Above described hybrids, made from 5'-end labeled 48 nt RNA oligonucleotide RNA1 and preformed specific circular DNA molecules - padlock probes PP1 and PP2, complementary to RNA1 molecule at 5'- end and center region (see Fig.6, parts C and D, respectively) were chosen as model objects for initial experiments. To verify reaction specificity control samples with no DNA probe (Fig. 6, part A) or samples with non specific padlock probe PP4, having no complementary region to RNA1 oligonucleotide (Fig. 6, part B) were used.

Experimental results indicate that, as expected, in control samples both in the presence and absence of dNTPs, only RNA degradation products were observed (Fig. 6, parts A and B). In opposite, in case of specific probe hybridization Phi29 polymerase degraded RNA up to the beginning of the double stranded RNA-DNA region, forming labeled RNA hydrolysis products, which in the presence of dNTPs were converted into labeled RCA product (Fig. 6, parts C-E). Formation of long labeled RCA product in the presence of dNTPs obviously indicates that synthesized RCA product comprises labeled RNA fragment at its 5'- end. These data prove the hypothesis that RNA hydrolysis products act as primers for RCA reaction.

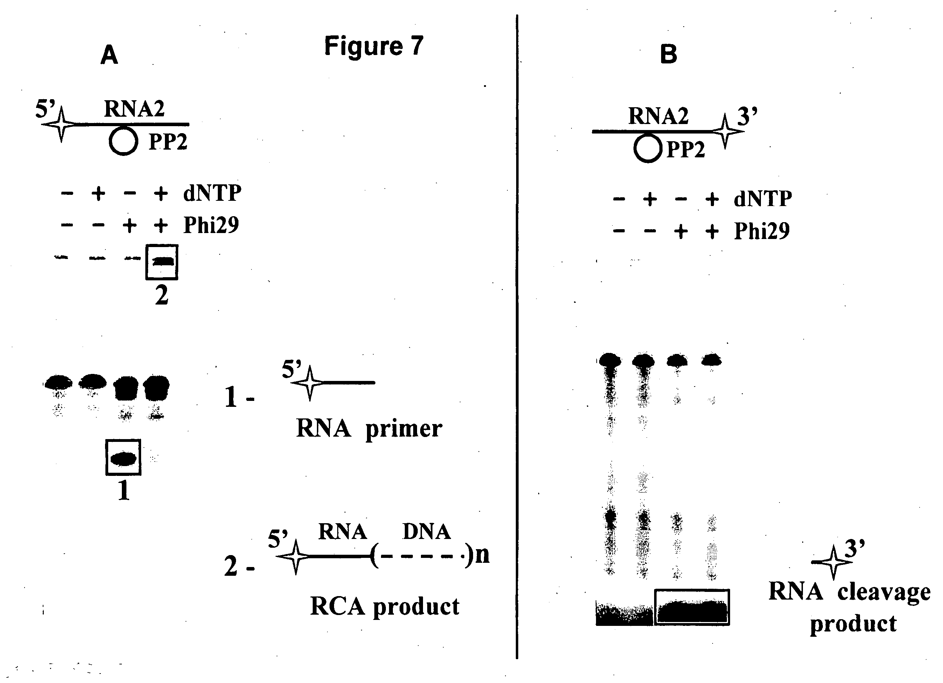

In order to prove that Phi29 polymerase synthesizes DNA after RNA processing, but not after accidental formation of double stranded structure between RNA molecule end and specific padlock probe, analogous experiments were performed in the presence and absence of dNTPs with 111 nt RNA oligonucleotide RNA2, labeled at either 5'- or 3'- ends and preformed specific padlock probe PP2 hybrids (see Fig. 7, parts A and B, respectively).

Experimental result confirmed that, like in case of RNA1 target (Fig. 6, parts C, D), Phi29 polymerase hydrolyzed RNA in 5'- end labeled RNA2 - PP2 hybrid forming smaller product than the substrate, while in the presence of dNTPs enzyme synthesized labeled RCA product (Fig. 7, part A). Different results were obtained in case of 3'- end labeled RNA2 target - PP2 hybrid. RNA degradation by Phi29 polymerase was observed, but no labeled RCA product was detected in the presence of dNTPs (Fig. 7, part B). These data confirm the presumption that Phi29 polymerase after processing of RNA 3'-end performs synthesis of RCA product, hence, it converts target RNA into primer for RCA product synthesis.

### 2.2. Conversion of target RNA into primer for DNA synthesis by other DNA polymerases

The experimental detail for this section is provided as "Example 8" below.

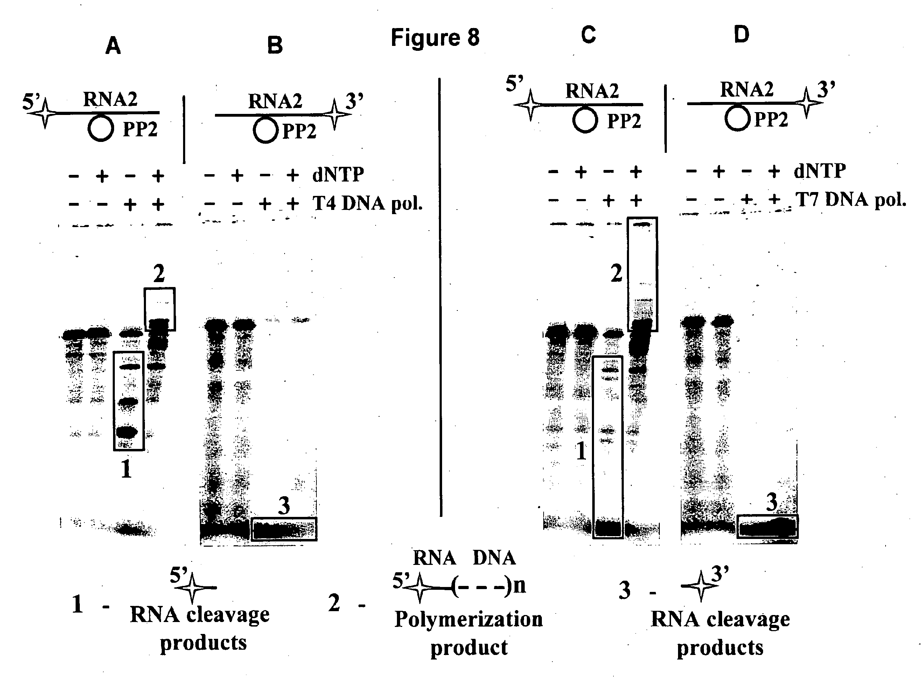

3'-5' exoribonucleolytic activity of Phi29 polymerase is not unique. Our biochemical studies of T4 and T7 DNA polymerases (see Fig. 8, A, B and C, D parts, respectively), show that other poymerases possessing deoxyribonuclease (exo+) activity can hydrolyze RNA in RNA-DNA hybrids and initiate DNA synthesis, however, RNase and strand displacement polymerase activities may have some peculiarities, e.g. in the absence of dNTPs main products of RNA hydrolysis by T7 DNA polymerase are nucleotides or short oligonucleotides, hence, contrary to Phi29 polymerase, T7 does not stop at RNA-DNA hybrid region (Fig. 8, part A). Since in the presence of dNTPs T7 DNA polymerase produced longer 5'-end labeled molecule than the initial RNA target, it can be concluded that in the presence of dNTPs this polymerase also processes RNA possibly to hybridization region followed by DNA synthesis. Of note, both tested polymerases showed significantly lower DNA synthesis efficiency in this system, resulting in shorter and less abundant synthesis product as compared with Phi29 polymerase (Fig. 8, parts A, C and Fig. 7, part A, respectively).

### 2.3. Positive factors for target RNA conversion into RCA primers

The experimental detail for this section is provided as "Example 9" to "Example 13" below.

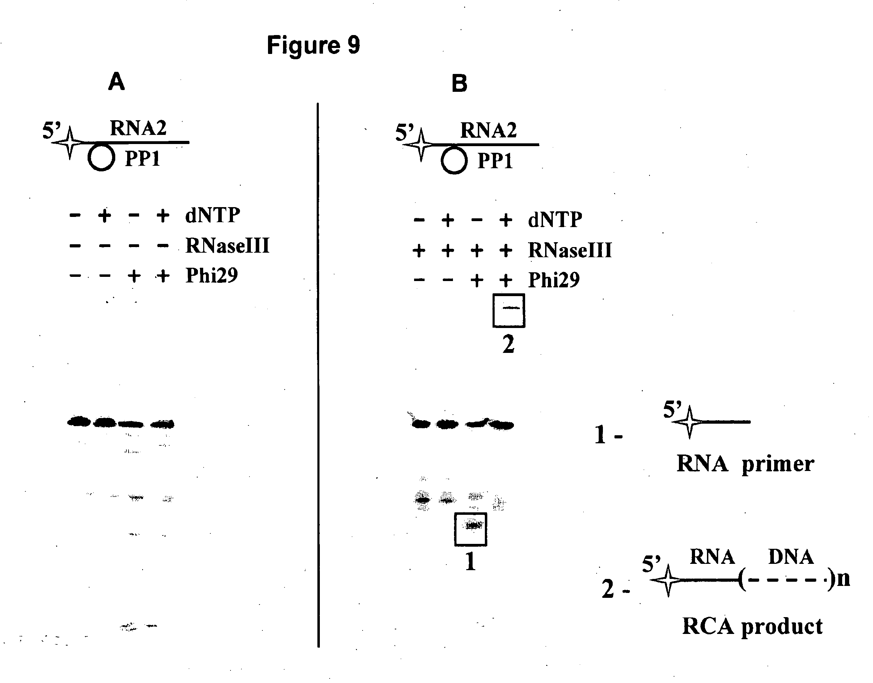

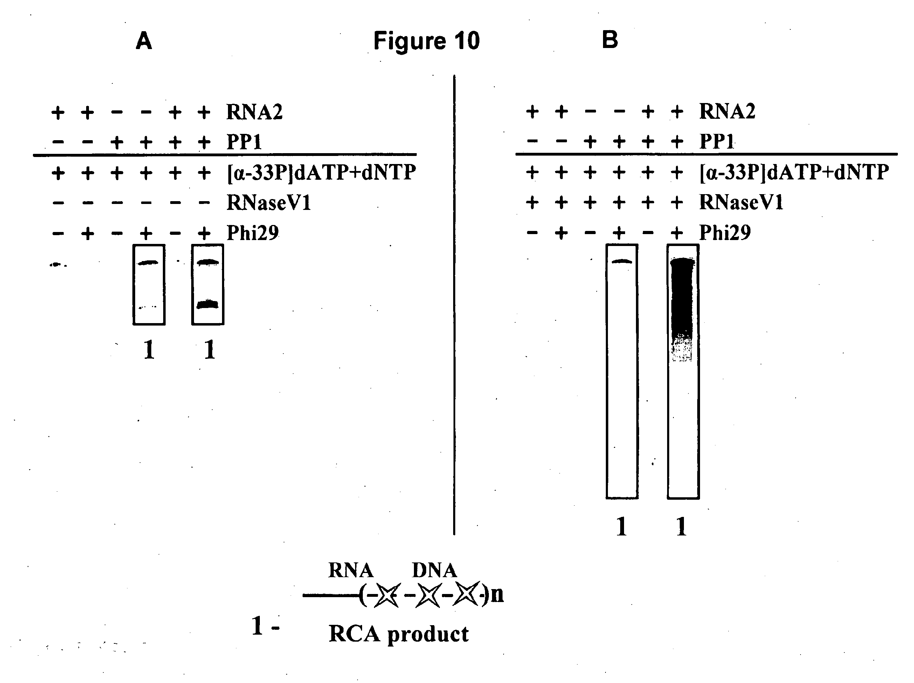

In some cases, such as experiments testing RNA2-preformed PP1 hybrids, neither RNA hydrolysis products, nor specific RCA product were obtained at standard reaction conditions using only Phi29 polymerase (Fig. 9, part A). In such cases the problem can be solved by additional RNA degradation with RNases. For example, addition of RNase III, which can hydrolyze regions of double stranded RNA (March and Gonzalez, 1990) resulted in the formation of both labeled RNA primers and labeled RCA product when testing the same RNA2-preformed PP1 hybrids (Fig. 9, part B). Addition of other RNase, RNase VI, which efficiently hydrolyzes shorter than 11 bp RNA regions (Lowman ir Draper, 1986) also significantly increased the yield of RCA product as detected by incorporation of labeled nucleotide during DNA synthesis (Fig. 10).

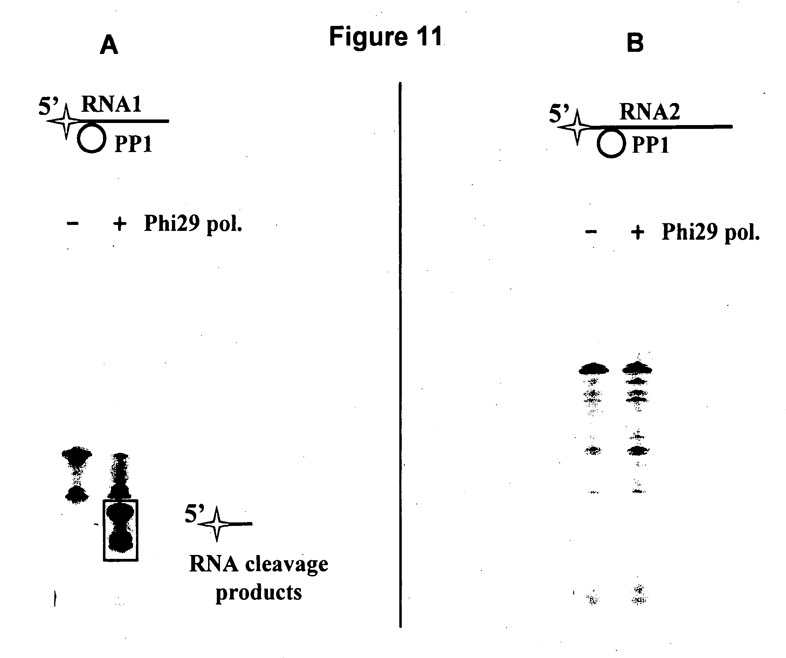

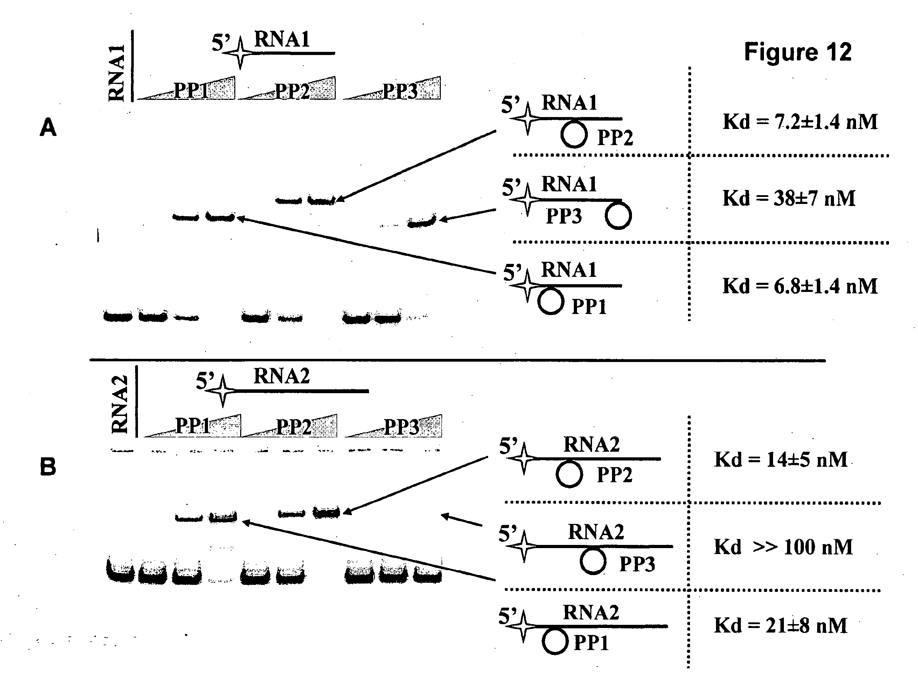

Effect of RNases can be explained in two ways:
1. Long RNA molecules can be inaccessible for Phi29 polymerase due to the existing intramolecular RNA-RNA structures at 3'-end of the target. This indirectly is confirmed by results of hydrolysis of both 111 nt RNA oligonucleotide RNA2 and its smaller portion - 48 nt oligonucleotide RNA1, hybrids with padlock probe PP1. It was shown that Phi29 polymerase degrades shorter oligonucleotide RNA1 when hybridized with DNA (Fig. 11, part A), while no detectable nuclease activity was found in case of longer RNA2, which likely could form double stranded RNA-RNA structures at 3' end of the molecule (Fig. 11, part B). This is in good agreement with data obtained on DNA-RNA hybrids hydrolysis, where Phi29 polymerase was inefficient when degrading long transcripts.
2. On the other hand, secondary double stranded RNA structures formed in long RNA molecules are not favorable for RNA-DNA hybrid formation. Electrophoretic mobility shift assay (EMSA) experiments comparing hybridization of 111 nt RNA oligonucleotide RNA2 and its portion - 48 nt oligonucleotide RNA1 with padlock probes PP1, PP2 and PP3, show that shorter RNA hybridizes with DNA more efficiently (see Fig. 12, parts A and B, respectively). Hence, it can be postulated that in addition to direct RNase action during RNA target processing, these enzymes may contribute to the more efficient formation of RNA-DNA hybrids.

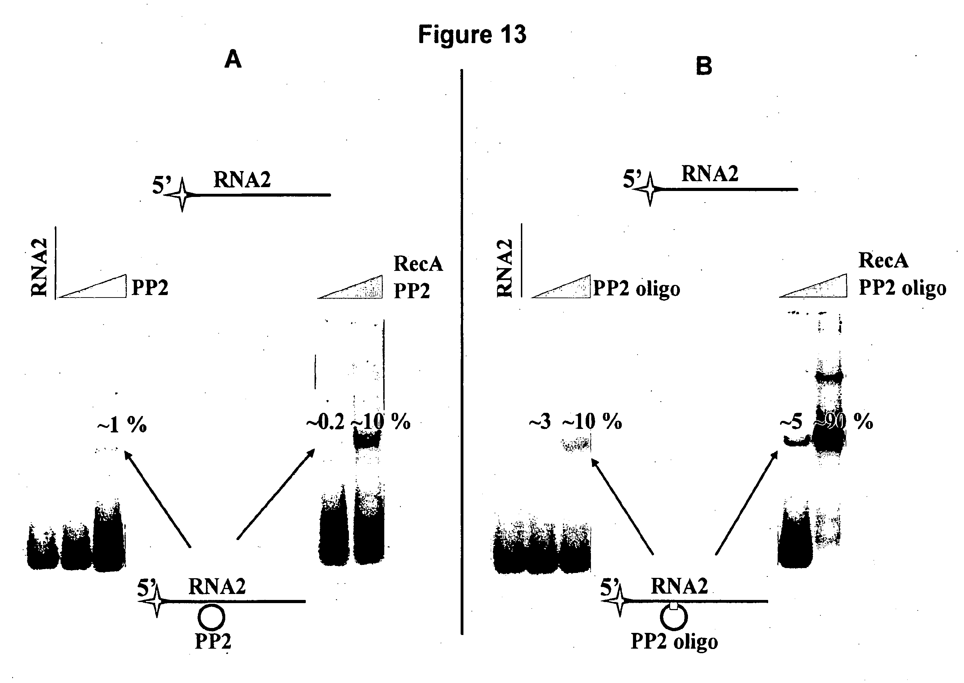

Another positive factor in RNA target conversion into primer could be RecA protein. There are some publications indicating that this protein promotes the formation of RNA-DNA hybrids (Kirkpatrick et al, 1992). Our EMSA experiments indicate that RecA promotes the formation of 111 nt RNA oligonucleotide RNA2 hybrid with preformed (circular) padlock probe PP2 (Fig. 13, part A), and especially with padlock probe PP2 precursor linear unligated oligonucleotide (Fig. 13, part B). This phenomenon may be important in various applications when hybridizing linear DNA oligonucleotides with various RNA targets prior to PP oligonucleotide ligation on target template.

### 3. Detection of RNA targets in RNA mixtures in vitro using preformed padlock probes

### 3.1. Specific detection of target RNA in RNA mixtures using padlock probes

Literature data indicate that DNA ligases, such as T4 DNA ligase, in addition to conventional DNA substrates can catalyze the ligation of nicked DNA involved in double stranded DNA-RNA structures (Nilsson *et al.,* 2001, Cristian *et al.,* 2001).

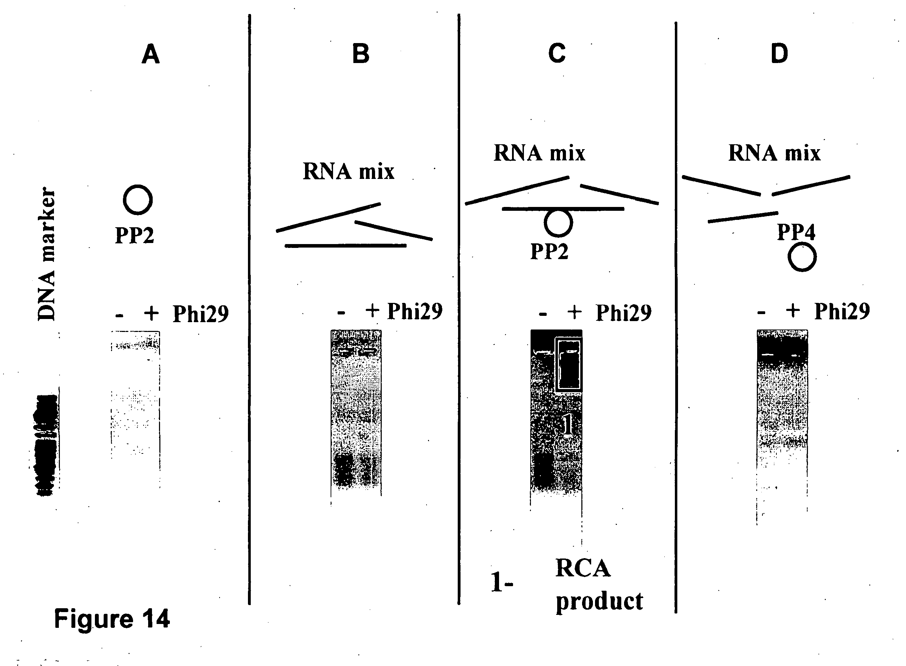

In order to test that our method is suitable for detection of individual RNA transcripts from complex RNA mixtures Phi29 catalyzed RCA reaction was performed using RNA mixture - RNA LR Ladder (Fermentas), one fragment of which (111 nt RNA oligonucleotide RNA2) had region complementary to specific padlock probe PP2. The experimental detail for this section is provided as "Example 14" below. It was shown that all control samples containing either only circular DNA molecule, or only padlock probe PP2 (Fig. 14, part A), or only RNA mixture (Fig. 14, part B), or RNA mixture with non specific non complementary padlock probe PP4 (Fig. 14, part D), generated no RCA product. In contrary, sample containing RNA mixture with specific padlock probe PP2 generated significant amounts of RCA product (Fig. 14, part C).

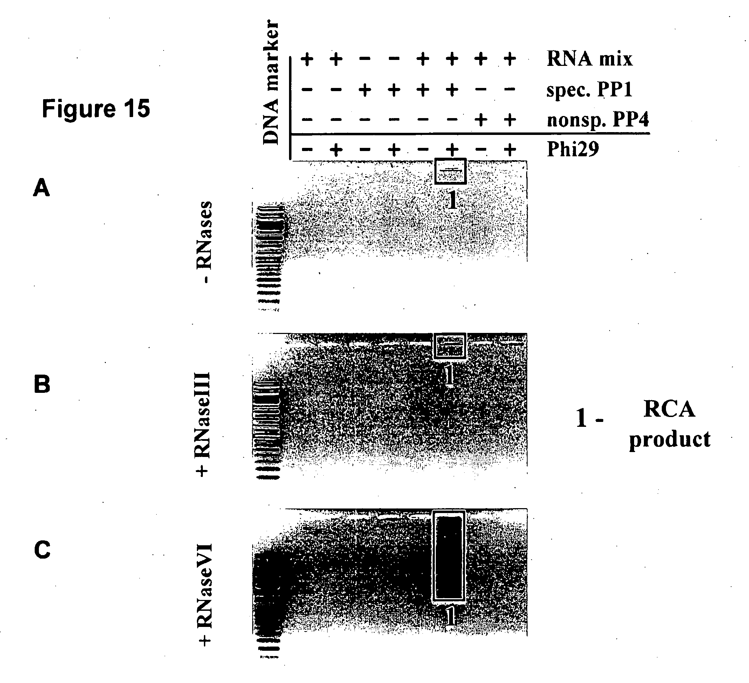

Addition of RNases, as described above, increased the yield of RCA product (see Fig. 15, parts A, B and C, respectively). Experimental detail is provided as "Example 15" below.

### 4. Detection of RNA targets in vitro by forming padlock probes by DNA ligation on RNA target

### 4.1. Ligation-based GAPDH transcript detection

Possibilities of novel detection method of this invention were tested in RCA with padlock probe precursors converting them into padlock probes by ligation of 3' and 5' ends of complementary oligonucleotide directly after hybridization with RNA template. The experimental detail is provided as "Example 16" below.

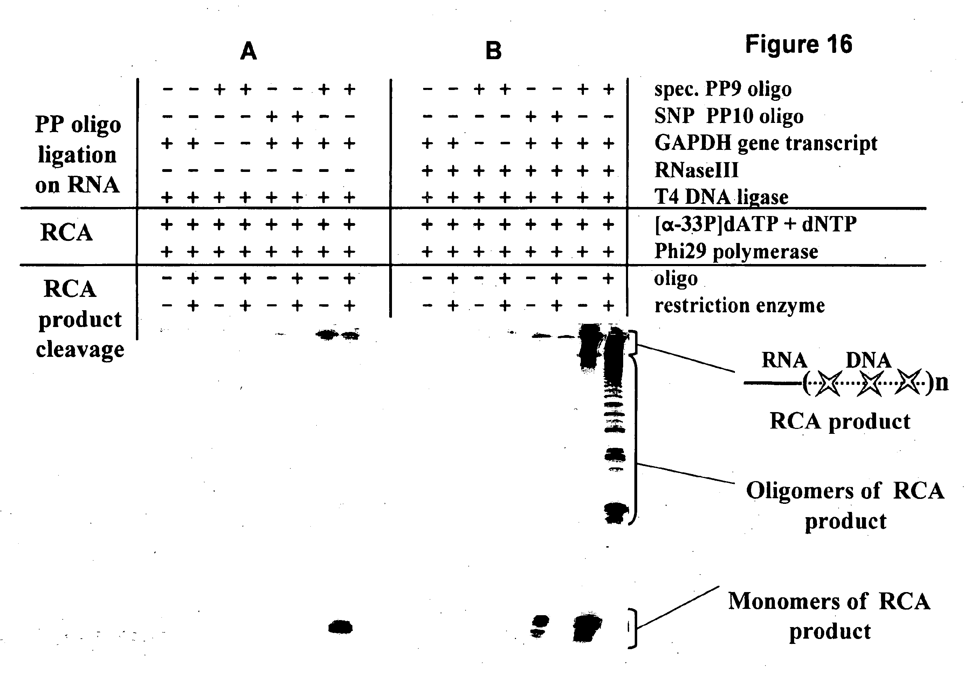

Experiments with GAPDH transcript revealed possibility of ligation - based on RNA target *in vitro* detection (Fig. 16). In absence of RNases specific RCA product were obtained when PP oligonucleotide was complementary to RNA target at ligation site (Fig. 16, part A)

It was shown that addition of RNase III significantly increased the yield of RCA product (Fig.16, part B). Most likely this relates to positive RNase III influence on DNA-RNA hybrid formation. However, presence of RNase III decreased reaction specificity. Trace of RCA product was observed using non-specific SNP PP10 oligonucleotides, which has non complementary 3' and 5' ends. This phenomenon may be related with ligation reaction specificity.

### 4.2 Ligation-based spliced β-globin transcript detection

Detection of RNA targets *in vitro* by forming padlock probes by DNA ligation on RNA target could be used in RNA sequence specificity, including single nucleotide polymorphism, deletion and insertion, analysis.

The method was applied in ligation-based spliced β-globin transcript detection. RCA experiments were performed with β globin pre-m RNA transcript before and after splicing. Splicing reaction was carried out as described in Example 17 and confirmed by RT PCR control experiments. Specificity of obtained RCA product was verified by hybridizing it with additional specific DNA oligonucleotides followed by restriction endonuclease digestion.

Our RCA data confirmed splicing phenomenon of β globin pre-m RNA transcript. It was shown specific RCA product was obtained only in the case of spliced β globin pre-m RNA transcript in presence of specific PP 11 oligonucleotide.

### 5. Detection of RNA targets in RNA mixtures in vitro by forming padlock probes by DNA ligation on RNA target

### 5.1. Detection of RNA targets in RNA mixtures

Possibilities of novel detection method of this invention were tested in RCA with RNA target in RNA mixture. Experimental detail is provided as "Example 18" below.

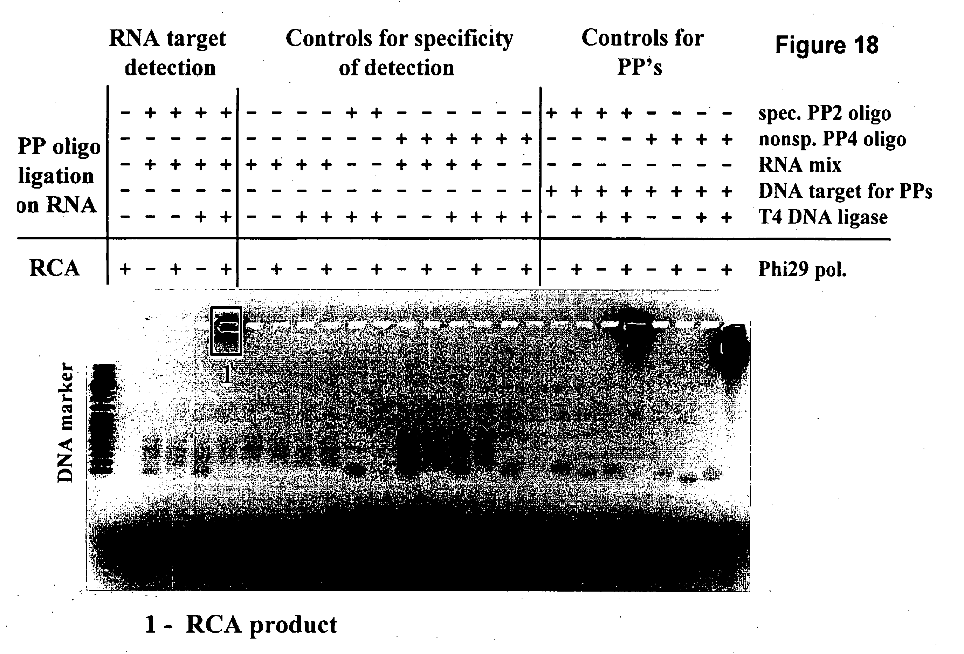

Our data show that sample containing linear specific padlock probe PP2 precursor oligonucleotide and RNA mixture, RNA LR Ladder, one fragment of which was complementary to PP2, generated RCA product (Fig. 18). All control samples, however, were negative.

### 5.2. Detection of GAPDH transcript in total mRNA from human HELA cells in vitro

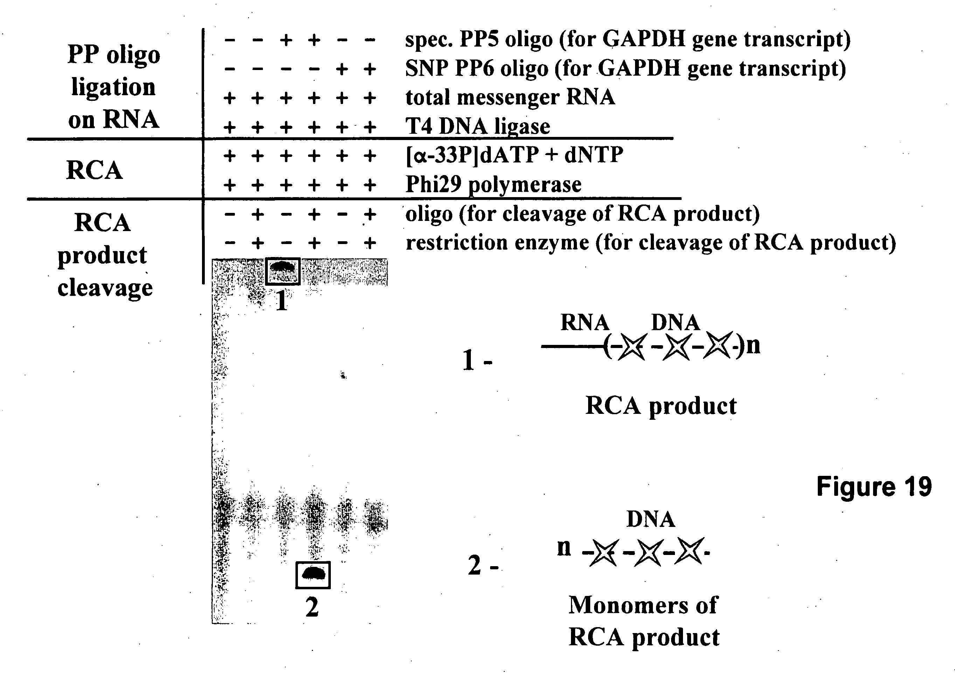

All previous data indicate that invented method could be used for detection of individual transcripts in total mRNA *in vitro* (Fig. 19). We have used this method to detect GAPDH transcript from total mRNA isolated from human HELA cells. The experimental detail is provided as "Example 19" below. Linear specific padlock probe PP5 precursor oligonucleotide was used, 5'- and 3'- ends of which were designed as complementary to GAPDH transcript. After padlock probe formation via ligation of hybridized precursor onto target RNA, RCA reaction was performed and specific RCA product was detected by incorporation on the labeled nucleotide into nascent RCA product. Parallel RT PCR control experiments confirmed the presence of detected transcript in total mRNA substrate and the absence of GAPDH encoding gene in iRNA preparation. Specificity of obtained RCA product was verified by hybridizing it with additional specific DNA oligonucleotides followed by restriction endonuclease digestion. Non specific (SNP) oligonucleotide differing in few nucleotides in ligation junction region did not generate any labeled RCA product.

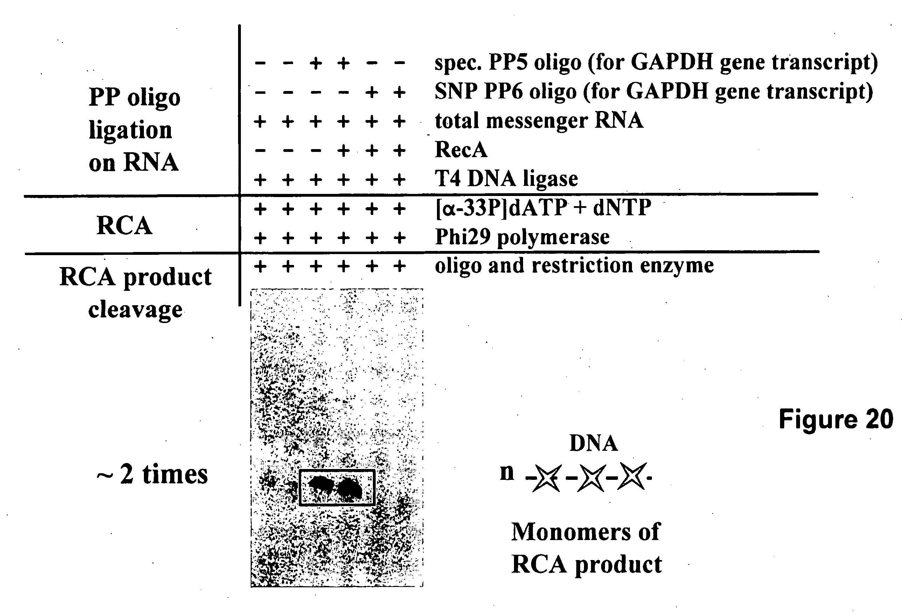

It was shown that in case of GAPDH transcript detection *in vitro* RecA protein increased the yield of RCA product several times (Fig. 20). Experimental detail is provided as "Example 20" below. Most likely this relates to positive RecA influence on DNA-RNA hybrid formation.

### 6. Detection of RNA targets in situ

### 6.1.Detection of GAPDH and ACTB transcripts in single human HELA cells

The experimental detail for this section is provided as "Example 20" to "Example 23" below.

One of the main directions of modem diagnostics is development of methods allowing to detect single molecule in the cell. The method of our invention was tested for suitability to use *in situ.*

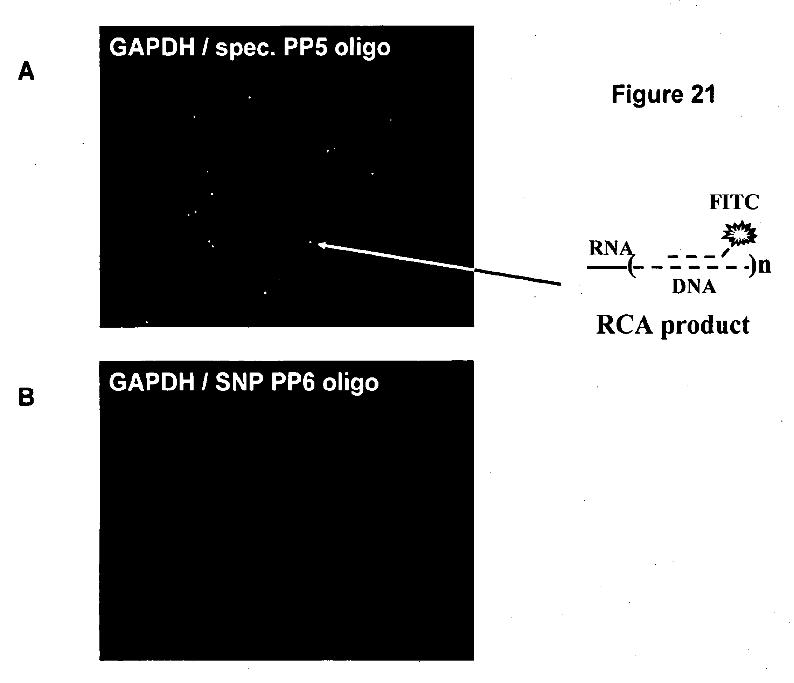

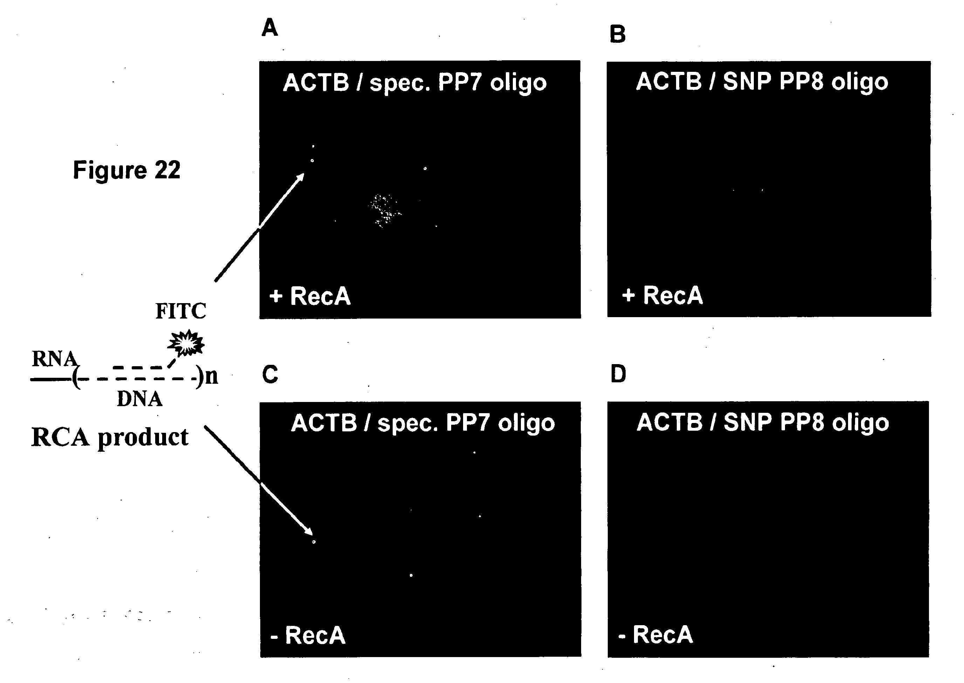

Obtained experimental results show that our method successfully and specifically detected both GAPDH and ACTB transcripts in human HELA cells cytoplasm (see Fig. 21, 22, respectively). Using specific padlock probe PP5 and PP7 precursor oligonucleotides for RCA and FITC labeled hybridization probes, we have detected the accumulates of fluorescent label in the cell cytoplasm, which indicated the localization of specific RCA product (see Fig. 21, part A and Fig. 22, parts A and C, respectively). Control samples with non specific (SNP) padlock probe PP6 and PP8 precursor oligonucleotides having few mismatched nucleotides at 3' and 5' ends did not generate any specific RCA product (see Fig. 21, part B and Fig. 22, parts B and D, respectively).

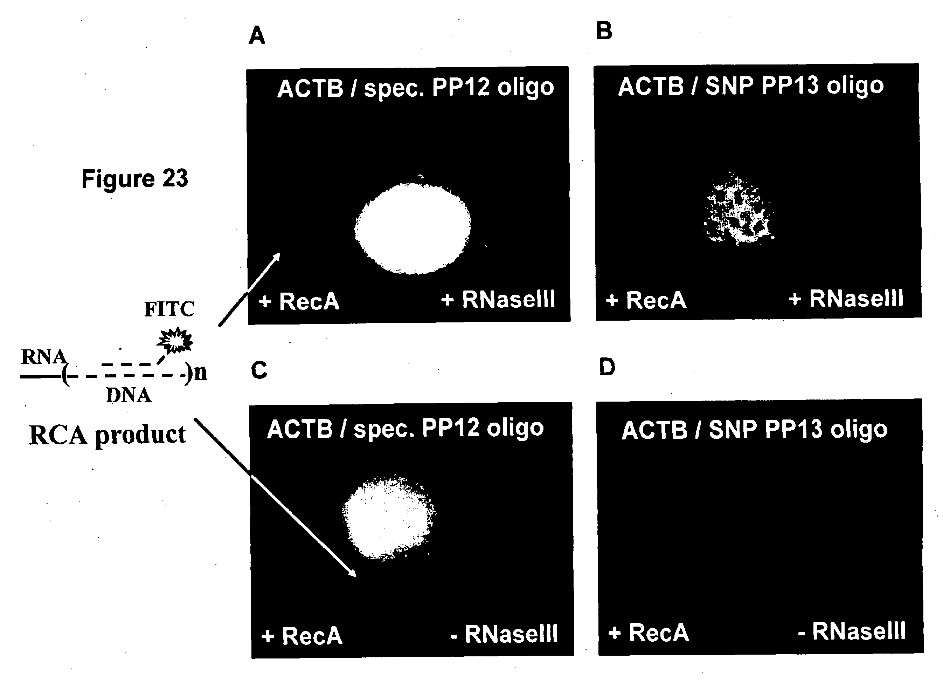

Noteworthy that, like in case of RNA transcript detection *in vitro,* addition of RecA protein increased the detection of ACTB transcript at least several times (see Fig. 22, parts A and C, respectively). Similar effect was observed in the presence of RNase (see Fig. 23, parts A and C, respectively).

### EXPERIMENTAL DETAIL

### Example 1 (Figure 1)

### Phi29 DNA polymerase RNase activity assay.

RNase activity of Phi29 polymerase was assayed using [³H]polyA substrate.

[8-³H] adenylic acid sodium salt (specific radioactivity is 604 mCi/mmol) was purchased from Amersham Biosciences. polyA (Pharmacia) was added to a final concentrations of 0.24 mM (specific radioactivity is 43 mCi/mmol) in DEPC-treated water.

Activity was measured in reaction mixtures of the following composition in DEPC-treated water: 1xTango™, 0.024 mM polyA (prepared as described above), 25 U Phi29 DNA polymerase or 25 U Phi29 (exo-) DNA polymerase, or without any enzyme (all components, except [³H]polyA, were from Fermentas)]. Samples were incubated at 37°C for 3 hours. After incubation, BSA and trichloracetic acid (TCA) were added into each sample to final concentrations of 2.5 mg/ml and 5%, respectively and left on ice for 10 min. To evaluate total radioactivity of the samples without enzymes, TCA in samples was replaced with distilled water. Following sample centrifugation at 13.2x1000 rpm for 10 min radioactivity of TCA-soluble fraction of supernatants was estimated. Sample aliquots were placed into scintillation vials with scintillation fluid and counted with liquid scintillation counter (Beckman).

The RNase activity in samples was evaluated by measuring amount (%) of acid soluble product. Each value calculated represents an average of two independent experiments.

### Example 2 (Figure2)

### A. Assay of Phi29 DNA polymerase RNase activity in Tris-acetate buffer in the presence of different cofactors

Activity assay was performed the same way as described in Example 1, except for reaction mixture composition The reaction mixture contained Tris-acetate buffer (1xTango™), 0.024 mM [³H]polyA, MgCl₂, MnCl₂, CoCl₂ at 10 mM final concentrations, or without any divalent metal ions (control sample), 25 U Phi29 DNA polymerase or no enzyme (control samples).

### B. Assay of Phi29 DNA polymerase RNase activity in Gly-KOH buffer in the presence of different cofactors

Activity assay was performed the same way as described in Example 1, except for reaction mixture composition. The reaction mixture contained Gly-KOH buffer (10 mM Glycine, 200 mM KCl, 0.1 mg/ml BSA, pH 9.0), 0.024 mM [³H]polyA, MgCl₂, MnCl₂, CoCl₂ at 10 mM final concentrations, or without any divalent metal ions (control sample), 25 U Phi29 DNA or without the enzyme (control samples).

### Example 3 (Figure 3)

Kinetic studies of RNA cleavage were carried out with single stranded 5'- end (part A) and 3'-end (part B) labeled RNA oligonucleotides (RNA1).

### A. Kinetic studies of 5'-end labeled RNA1 cleavage by Phi29 DNA polymerase

5'-end labeling of RNA1 was carried out using KinaseMax™ kit (Ambion) following manufacturer's recommendations, at a final concentration of 1 µM in 20 µl of reaction mixture [60 µCi [γ³³P]ATP, 1×T4 PNK buffer, 10 U T4 PNK in nuclease-free water (all components, except [γ³³P]ATP (Amersham Biosciences), were from Ambion)] for 1 hour at 37°C. After the incubation the reaction mixture was heated for 10 minutes at 70°C and placed on ice.

Before cleavage RNA1 was diluted to 4 nM in 30 µl with 1×Tango™ buffer in DEPC-treated water (Fermentas), heated for 3 minutes at 65°C, cooled to room temperature for 10 minutes and placed on ice. For cleavage reaction RNA1 was diluted to 1 nM concentration in 49.25 µl of reaction mixture [1×Tango™, 1 U/µl Ribolock™ ribonuclease inhibitor in DEPC-treated water (all components were from Fermentas)] and pre-warmed at 37°. An 8 µl aliquot was removed and mixed with 8 µl of 2xLoading dye solution (Fermentas) on ice before the addition of an enzyme. Following the addition of 6.3 U Phi29 DNA polymerase (Fermentas) to the remaining reaction mixture, aliquots were removed at various times (0.5, 2, 10, 120 minutes, respectively) and mixed with 8 µl of 2×Loading dye solution on ice. All aliquots were heated for 10 minutes at 70°C and placed on ice.

1 µl of 5'-end labeled samples were loaded onto 8% denaturing acrylamide gel [8% acrylamide/bisacrylamide (29:1), 7 M urea, 1×TBE (Fermentas)] and subjected to electrophoresis on Macrophor sequencing station (Pharmacia) at 2000 V with cooling for 2 hours (gel parameters: length 53 cm, thickness 0.2 mm). After electrophoresis, gel was dried and analyzed using BAS-MS Imaging Plates (Fujifilm), Cyclone Storage Phosphor System and OptiQuant™ Image Analysis software (Packard Instruments).

### B. Kinetic studies of 3'-end labeled RNA1 cleavage by Phi29 DNA polymerase

3'-end labeling of RNA1 was carried out at a final concentration of 1 µM in 10 µl of reaction mixture [30 µCi [³²P]pCp, 1 mM ATP, 1×T4 RNA ligase buffer, 10 µg/ml BSA, 10 U T4 RNA ligase in DEPC-treated water (all components, except [³²P]pCp (Amersham Biosciences), were from Fermentas)] overnight (~16 hours) at +4°C. Following incubation the reaction mix was heated for 10 minutes at 70°C and placed on ice.

In the following step 3'-end labeled RNA1 was 3'-dephosphorylated using T4 PNK: 1 µl of 3'-end labeled RNA1 was incubated in reaction mixture [1×Tango™, 1 U/µl Ribolock™ ribonuclease inhibitor, 10 U T4 PNK in DEPC-treated water (all components were from Fermentas)] for 30 minutes at 37°C. Afterwards reaction mixture was heated for 10 minutes at 70°C and placed on ice.

Before cleavage RNA1 was diluted to 4 nM in 15 µl with 1×Tango™ buffer (Fermentas), heated for 3 minutes at 65°C, cooled to room temperature for 10 minutes and placed on ice. Cleavage reaction with Phi29 DNA polymerase was performed as described in Example 3, part A.

2 µl of samples were loaded onto 8% denaturing acrylamide gel and run on adjustable slab gel system (Cole-Parmer) at 250 V at room temperature for 2 hours (gel parameters: length 20 cm, thickness 0.75 mm). Gel was dried and analyzed as described in Example 3, part A.

### Example 4 (Figure 4)

Kinetic studies of RNA-padlock probe (PP) hybrids cleavage by Phi29 DNA polymerase were carried out with 5'- end (parts A-C) and 3'-end (parts D-F) labeled RNA oligonucleotide (RNA1) hybrids with various preformed padlock probes.

### A. Kinetic studies of 5'-end labeled RNA1-PP1 hybrid cleavage by Phi29 DNA polymerase

5'-end labeling of RNA1 was performed as described in Example 3, part A.

Phosporylated PP1 DNA oligonucleotide was circularized on an appropriate DNA oligonucleotide (PL1 oligo), which later was removed by T7 DNA polymerase exonucleolytic activity.

PP1 oligonucleotide was 5'-phosphorylated at a final concentration of 10 µM in 10 µl of reaction mixture [1×Tango™, 50 µM ATP, 5 U T4 PNK in DEPC-treated water (all components were from Fermentas)] for 30 minutes at 37°C. Following the incubation the reaction mixture was heated for 10 minutes at 70°C and placed on ice.

10 µl of phosphorylated PP1 oligonucleotide were used for hybridization and ligation on PL1 oligonucleotide reactions. The final ligation reaction mixture volume was 500 µl [200 nM PP1, 200 nM PL1, 1×Tango™, 1 mM ATP, 75 U T4 DNA ligase in DEPC-treated water (buffer, ATP and the enzymes were from Fermentas)]. The hybridization was performed in the absence of ATP and T4 DNA ligase (which were added later) by placing the sample into boiling water bath and allowing to cool to room temperature for 2 hours. After cooling, ATP and T4 DNA ligase were added to the reaction mixture and ligation was performed for 1 hour at 37°C. After incubation, sample was placed again into a boiling water bath and allowed to cool to room temperature for 2 hours. T4 DNA ligase was inactivated during this step and simultaneously PP1 re-hybridized to PL1. After re-hybridization, PP1-PL1 hybrid was treated with T7 DNA polymerase (10 U for 1 µg DNA) (Fermentas) for 1 hour at 37°C, afterwards T7 DNA polymerase was inactivated by heating the sample for 10 minutes at 70°C.

For RNA-DNA hybridization RNA1 and PP1 were mixed at final concentration of 4 nM and 40 nM, respectively, in 15 µl of hybridization mixture with 1×Tango™ buffer in DEPC-treated water (both components were from Fermentas), heated for 3 minutes at 65°C, cooled to room temperature for 10 minutes and placed on ice.

Cleavage reaction was performed as described in Example 3, part A, except for cleavage substrate, which was RNA1-PP1 hybrid (final concentrations of RNA1 and PP1 were 1 nM and 10 nM, respectively). Electrophoresis and gel analysis were carried out as described in Example 3, part A.

### B. Kinetic studies of 5'-end labeled RNA1-PP2 hybrid cleavage by Phi29 DNA polymerase

All manipulations were performed exactly as described in Example 4, part A, except for cleavage substrate, which was RNA1-PP2 hybrid. PP2 was prepared exactly as PP1 described in Example 6, part A, only PP2 oligonucleotide was circularized on PL2 oligonucleotide.

### C. Kinetic studies of 5'-end labeled RNA1-PP3 hybrid cleavage by Phi29 DNA polymerase

All manipulations were performed exactly as described in Example 4, part A, except for cleavage substrate, which was RNA1-PP3 hybrid. PP3 was prepared exactly as PP1 described in Example 6, part A, only PP3 oligonucleotide was circularized on PL3 oligonucleotide.

### D. Kinetic studies of 3'-end labeled RNA1-PP1 hybrid cleavage by Phi29 DNA polymerase.

3'-end labeling and 3'-dephosphorylation of RNA1 were performed as described in Example 3, part B.

Preparation of PP1, RNA1-PP1 hybrid formation and it's cleavage by Phi29 DNA polymerase were performed as described in Example 4, part A. Electrophoresis and gel analysis were carried out as described in Example 3, part B.

### E. Kinetic studies of 3'-end labeled RNA1-PP2 hybrid cleavage by Phi29 DNA polymerase.

3'-end labeling and 3'-dephosphorylation of RNA1 were performed as described in Example 3, part B.

Preparation of PP1, RNA1-PP2 hybrid formation and it's cleavage by Phi29 DNA polymerase were performed as described in Example 4, part B. Electrophoresis and gel analysis were carried out as described in Example 3, part B.

### F. Kinetic studies of 3'-end labeled RNA1-PP3 hybrid cleavage by Phi29 DNA polymerase.

3'-end labeling and dephosphorylation of RNA1 were performed as described in Example 5, part B.

Preparation of PP3, RNA1-PP3 hybrid formation and it's cleavage by Phi29 DNA polymerase were performed as described in Example 4, part C. Electrophoresis and gel analysis were carried out as described in Example 3, part B.

### Example 5 (Figure 5)

### Determination of final RNA digestion (by Phi29 DNA polymerase) products

RNA1 and RNA1-PP cleavage was performed with Phi29 DNA polymerase. RNA digestion by Phi29 DNA polymerase products were determined by comparison 5'-labeled RNA cleavage pattern with the same labeled RNA alkaline hydrolysis ladder and RNaseT1 sequencing marker.

5'-end labeling of RNA1 was performed as described in Example 3, part A. Padlock probes PP1, PP2, PP3 and their hybrids RNA1-PP1, RNA1-PP3, RNA1-PP3 were prepared as described in Example 4, part A, B and C respectively.

Cleavage reactions with Phi29 DNA polymerase were performed for 2 hours at 37°C in 20 µl of reaction mixture containing: 1 nM RNA1 (or various RNA1-PP hybrids; final concentrations of RNA1 and PP were 1 nM and 10 nM, respectively), 1×Tango™, 1 U/µl Ribolock™ ribonuclease inhibitor, 3 U Phi29 DNA polymerase in DEPC-treated water (the buffer and the enzymes were from Fermentas). Following incubation 20 µl of 2×Loading dye solution (Fermentas) were added to stop the reaction. Samples were heated for 10 minutes at 70°C and placed on ice before loading onto acrylamide gel.

Preparation of RNA1 alkaline hydrolysis ladder: 2 µl of 5'-end labeled RNA1 (1 µM) were mixed with 3 µl of yeast RNA (1 mg/ml), followed by the addition of 10 µl of alkaline hydrolysis buffer (50 mM sodium carbonate pH 9.2, 1 mM EDTA) to the RNA mixture placed on ice. The sample was heated at 95°C for 2 minutes and immediately placed on ice, afterwards equal volume of 2×Loading dye solution (Fermentas) was added.

Preparation of RNA1 RNaseT1 sequencing marker: 2 µl of 5'-end labeled RNA1 (1 µM) were mixed with 3 µl of yeast RNA (1 mg/ml), followed by the addition of 10 µl of RNA sequencing buffer (20 mM sodium citrate pH 5.0, 7 M urea, 1 mM EDTA) to the RNA mixture placed on ice. The mixture was heated at 50°C for 5 minutes and cooled to room temperature, afterwards 3 µl of RNaseT1 (0.2 U/µl) (Fermentas) were added. The sample was incubated for 15 minutes at room temperature, followed by the phenol/chloroform (5:1, pH 4.5) extraction and addition of equal volume of 2×Loading dye solution (Fermentas) on ice.

Preparation of RNA1 ladders with 3'-terminal hydroxyls: RNA1 alkaline hydrolysis and RNaseT1 sequencing ladders were treated with T4 PNK, which can remove 2'-, 3'-, or 2',3'-cyclic phosphate. An aliquot of RNA1 ladder was EtOH precipitated: 1 µl of prepared RNA1 ladder (with 2×Loading dye) was mixed with 0.25 µl glycogen (20 mg/ml) (Fermentas), 0.5 µl sodium acetate (3 M, pH 5.2) and 5.25 µl EtOH, chilled at - 20°C for few hours and centrifuged at 16 000 × g for 15 minutes at +4°C. Pellet was washed with 70% EtOH and centrifuged at 16 000 × g for 15 minutes at +4°C, dried for 10 minutes at room temperature and dissolved in 5 µl of DEPC-treated water (Fermentas). The whole sample was diluted in reaction buffer [1×DNaseI buffer with MgCl₂, 1 U/µl Ribolock™ inhibitor, 6 U T4 PNK (all components were from Fermentas)] and incubated at 37°C for 30 minutes, followed by the addition of equal volume of 2×Loading dye (Fermentas) on ice.

1 µl of RNA1 and RNA1/PP hybrids cleavage samples (or RNA1 ladders) and 1.5 µl of RNA1 T4 PNK-treated ladders were loaded onto 8% denaturing acrylamide gel [8% acrylamide/bisacrylamide (29:1), 7 M urea, 1×TBE (Fermentas)]. Electrophoresis and gel analysis were carried out as described in Example 3, part A.

### Example 6 (Figure 6)

Phi29 DNA polymerase activity assay was performed on RNA1 (part A) and RNA1-PP hybrids (parts B-C) in the absence or presence of dNTP mix. The reaction substrate and products were traced by label at 5'-end of RNA1.

### A. Assay of Phi29 polymerase activity on RNA1

5'-end labeling of RNA1 was performed as described in Example 3, part A.

RNA1 for cleavage was prepared as described in Example 3, part B, except for the sample volume, which was 25 µl.

Incubation of 5'-end labeled RNA1 with Phi29 DNA polymerase was performed as described in Example 5, except that reaction was carried out in the presence or absence of 1 mM dNTP.

5 µl of 5'-end labeled samples were loaded onto 8% denaturing acrylamide gel, run and analyzed as described in Example 5, part B.

### B. Assay of Phi29 polymerase activity on RNA1 in the presence of nonspecific padlock-probe (PP4)

5'-end labeling of RNA1 was performed as described in Example 3, part A.

PP4 was prepared as described in Example 4, part A, only PP4 oligonucleotide was circularized on PL4 oligonucleotide.

Mixing of RNA1 with preformed PP4 was performed in the same conditions as described in Example 4, part A, except for the sample volume, which was 25 µl.

Incubation of 5'-end labeled RNA1 in the presence of PP4 with Phi29 DNA polymerase was performed as described in Example 5, except that reaction was carried out in the presence or absence of 1 mM dNTP.

5 µl of 5'-end labeled samples were loaded onto 8% denaturing acrylamide gel, run and analyzed as described in Example 3, part B.

### C. Assay of Phi29 polymerase activity on RNA1-PP1 hybrid

5'-end labeling of RNA1 was performed as described in Example 3, part A.

PP1 was prepared as described in Example 4, part A.

RNA-DNA hybrid formation was performed as described in Example 4, part A, except for the sample volume, which was 25 µl.

Incubation of 5'-end labeled RNA1-PP1 hybrid with Phi29 DNA polymerase was performed as described in Example 5, except that reaction was carried out in the presence or absence of 1 mM dNTP.

5 µl of 5'-end labeled samples were loaded onto 8% denaturing acrylamide gel, run and analyzed as described in Example 3, part B.

### D. Assay of Phi29 polymerase activity on RNA1-PP2 hybrid

5'-end labeling of RNA1 was performed as described in Example 4, part A.

PP2 was prepared as described in Example 4, part A, only PP2 oligonucleotide was circularized on PL2 oligonucleotide.

RNA-DNA hybrid formation was performed as described in Example 4, part A, except for the sample volume, which was 25 µl.

Incubation of 5'-end labeled RNA1-PP2 hybrid with Phi29 DNA polymerase was performed as described in Example 5, except that reaction was carried out in the presence or absence of 1 mM dNTP.

5 µl of 5'-end labeled samples were loaded onto 8% denaturing acrylamide gel, run and analyzed as described in Example 3, part B.

### E. Assay of Phi29 polymerase activity on RNA1-PP3 hybrid

5'-end labeling of RNA1 was performed as described in Example 3, part A.

PP3 was prepared as described in Example 4, part A, only PP3 oligonucleotide was circularized on PL3 oligonucleotide.

RNA-DNA hybrid formation was performed as described in Example 4, part A, except for the sample volume, which was 25 µl.

Incubation of 5'-end labeled RNA1-PP3 hybrid with Phi29 DNA polymerase was performed as described in Example 5, except that reaction was carried out in the presence or absence of 1 mM dNTP.

5 µl of 5'-end labeled samples were loaded onto 8% denaturing acrylamide gel, run and analyzed as described in Example 3, part B.

### Example 7 (Figure 7)

Test for RNA conversion into a primer for RCA by Phi29 DNA polymerase was performed on RNA2-PP2 hybrid. The reaction substrate and products were traced by the label at 5'-end or 3'-end of RNA2.

### A. Test for RNA conversion into a primer by Phi29 DNA polymerase for RCA (using 5'-end labeled RNA)

5'-end labeling of RNA2 was performed using KinaseMax™ kit (Ambion) following manufacturers recommendations. 20 pmol of RNA2 was dephosphorylated with 0.1 U CIAP in 10 µl of 1×dephosphorylation buffer for 1 hour at 37°C, CIAP then was removed using phosphatase removing reagent. RNA2 was 5'-end labeled at a final concentration of 1 µM in 20 µl of reaction mixture [60 µCi [γ³³P]ATP, 1 xT4 PNK buffer, 10 U T4 PNK in nuclease-free water (all components except for [γ³³P]ATP (Amersham Biosciences), were from Ambion)] for 1 hour at 37°C. Following the incubation the reaction mixture was heated for 10 minutes at 70°C and placed on ice.

PP2 was prepared as PP1 as described in Example 4, part A, only PP2 oligonucleotide was circularized on PL2 oligonucleotide.

RNA-DNA hybridization was prepared as described in Example 4, part A, except for the sample volume, which was 25 µl, and hybridizing components, which were RNA2 and PP2, respectively.

Incubation of 5'-end labeled RNA2-PP2 hybrid with-Phi29 DNA polymerase was performed as described in Example 6, part D, except for the RNA-DNA substrate, which was RNA2-PP2 hybrid.

5 µl of 5'-end labeled samples were loaded onto 8% denaturing acrylamide gel, run and analyzed as described in Example 3, part B.

### B. Test for RNA conversion into a primer by Phi29 DNA polymerase for RCA (using 3'-end labeled RNA). 3'-end labeling and 3'-dephosphorylation of RNA2 were performed in the same way as described in Example 3, part B.

PP2 was prepared as PP1 as described in Example 4, part A, only PP2 oligonucleotide was circularized on PL2 oligonucleotide.

RNA-DNA hybridization and incubation with Phi29 DNA polymerase were prepared as described in Example 7, part A, except for the substrate, which was 3'-end labeled RNA2-PP2 hybrid.

2 µl of 3'-end labeled samples were loaded onto 8% denaturing acrylamide gel, run and analyzed as described in Example 3, part B.

### Example 8 (Figure 8)

Test for RNA conversion into a primer for RCA by other (exo+) DNA polymerases was performed with either T7 or T4 DNA polymerases on RNA2-PP2 hybrid. The reaction products were traced by labeling RNA2 at 5'- or 3'-end.

### A. Test for RNA conversion into a primer by T7 DNA polymerase for RCA (using 5'-end labeled RNA)

5'-end labeling of RNA2 was performed as described in Example 7, part A.

PP2 was prepared as PP1 as described in Example 4, part A, only PP2 oligonucleotide was circularized on PL2 oligonucleotide.

RNA-DNA hybridization was prepared as described in Example 4, part A, except for the sample volume, which was 45 µl, and hybridizing components, which were RNA2 and PP2, respectively.

Incubation with T7 DNA polymerase (Fermentas) was performed as described in Example 7, part A, except for that T7 DNA polymerase was used instead of Phi29 DNA polymerase.

5 µl of 5'-end labeled samples were loaded onto 8% denaturing acrylamide gel, run and analyzed as described in Example 3, part B.

### B. Test for RNA conversion into a primer by T7 DNA polymerase for RCA (using 3'-end labeled RNA)

3'-end labeling and 3'-dephosphorylation of RNA2 was performed in the same way as described in Example 3, part B.

PP2 was prepared as PP1 as described in Example 4, part A, only PP2 was circularized on PL2.

RNA-DNA hybridization and incubation with T7 DNA polymerase (Fermentas) were prepared as described in Example 8, part A, except for the substrate, which was 3'-end labeled RNA2-PP2 hybrid.

2 µl of 3'-end labeled samples were loaded onto 8% denaturing acrylamide gel, run and analyzed as described in Example 3, part B.

### C. Test for RNA conversion into a primer by T4 DNA polymerase for RCA (using 5'-end labeled RNA)

5'-end labeling of RNA2 was performed as described in Example 7, part A.

PP2 was prepared as PP1 as described in Example 4, part A, only PP2 oligonucleotide was circularized on PL2 oligonucleotide.

RNA-DNA hybridization was prepared as described in Example 8, part A.

Incubation with T4 DNA polymerase (Fermentas) was performed as described in Example 7, part A, except for that T4 DNA polymerase was used instead of Phi29 DNA polymerase.

5 µl of 5'-end labeled samples were loaded onto 8% denaturing acrylamide gel, run and analyzed as described in Example 3, part B.

### D. Test for RNA conversion into a primer by T4 DNA polymerase for RCA (using 3'-end labeled RNA)

3'-end labeling and 3'-dephosphorylation of RNA2 was performed in the same way as described in Example 3, part B.

PP2 was prepared as PP1 as described in Example 4, part A, only PP2 oligonucleotide was circularized on PL2 oligonucleotide.

RNA-DNA hybridization and incubation with T4 DNA polymerase (Fermentas) were prepared as described in Example 8, part C, except for the substrate, which was 3'-end labeled RNA2-PP2 hybrid.

2 µl of 3'-end labeled samples were loaded onto 8% denaturing acrylamide gel, run and analyzed as described in Example 3, part B.

### Example 9 (Figure 9)

Test for target RNA conversion into a primer for RCA by DNA polymerase in the presence of RNases that cleave double-stranded RNA, was performed with Phi29 DNA polymerase and RNaseIII on RNA2/PP hybrid. Reaction products were traced by labeling RNA2 at 5'-end.

### A. Test for RNA target conversion into a primer for RCA by Phi29 DNA polymerase in the absence of RNases, which cleave double-stranded RNA

5'-end labeling of RNA2 was performed as described in Example 7, part A.

PP1 was prepared as described in Example 4, part A.

RNA-DNA hybridization was prepared as described in Example 6, part A, only hybridization was performed in 45 µl and hybridizing components were RNA2 and PP2 respectively.

Incubation with Phi29 DNA polymerase was performed for 2 hours at 37°C in 10 µl reaction mixture containing: RNA2-PP1 hybrid (final concentrations of RNA2 and PP1 were 1 nM and 10 nM, respectively), 75 ng yeast RNA, 1×Tango™ in DEPC-treated water in the presence or absence of 1 mM dNTPs or 1.5 U Phi29 DNA polymerase (all components, except yeast RNA (Pharmacia), were from Fermentas). After incubation 10 µl of 2×Loading dye solution (Fermentas) were added to stop the reaction. Samples were heated for 10 minutes at 70°C and placed on ice before loading onto acrylamide gel.

5 µl of 5'-end labeled samples were loaded onto 8% denaturing acrylamide gel, run and analyzed as described in Example 3, part B.

### A. Test for RNA target conversion into a primer for RCA by Phi29 DNA polymerase in the presence RNaseIII

5'-end labeling of RNA2 was performed as described in Example 7, part A.

PP 1 was prepared as described in Example 4, part A.

RNA-DNA hybridization was prepared as described in Example 4, part A, only hybridization was performed in 45 µl and hybridizing components were RNA2 and PP2, respectively.

Incubation with Phi29 DNA polymerase was performed for 2 hours at 37°C in 10 µl reaction mixture containing: RNA2-PP1 hybrid (final concentrations of RNA2 and PP1 were 1 nM and 10 nM, respectively), 75 ng yeast RNA, 1×Tango™ (Fermentas), 8·10⁻³ U RNaseIII in DEPC-treated water in the presence or absence of 1 mM dNTPs or 1.5 U Phi29 DNA polymerase (all components, except yeast RNA (Pharmacia) and RNaseIII (Epicentre), were from Fermentas). All components were mixed on ice, following RNaseIII addition. After incubation 10 µl of 2×Loading dye solution (Fermentas) were added to stop the reaction. Samples were heated for 10 minutes at 70°C and placed on ice before loading onto acrylamide gel.

5 µl of 5'-end labeled samples were loaded onto 8% denaturing acrylamide gel, run and analyzed as described in Example 3, part B.

### Example 10 (Figure 10)

Test for increased efficiency of RCA (using RNA-PP hybrid) by double-stranded RNA hydrolyzing enzymes was also performed with Phi29 DNA polymerase in the presence of RNaseV1. Experiments were performed with unlabeled RNA2-PP1 hybrid. The reaction products were traced by incorporating [α³³P] dATP into RCA product.

### A. Test of RCA efficiency (in the absence of RNases, which cleave double-stranded RNA)

PP1 was prepared as described in Example 4, part A.

RNA-DNA hybridization was performed as described in Example 4, part A, only hybridization between unlabeled RNA2 and PP1 was performed at concentrations 40 nM and 4 nM, respectively. Hybridization volume was 25 µl.

RNA2 for cleavage reaction was prepared as RNA1 as described in Example 4, part A, only the volume was 25 µl and unlabeled RNA2 concentration was 40 nM.

PP 1 for cleavage reaction was prepared by diluting PP 1 to 4 nM in 25 µl with 1×tango™ buffer in DEPC-treated water (Fermentas), heating for 3 minutes at 65°C, cooling to room temperature for 10 minutes and placing on ice.

Incubation with Phi29 DNA polymerase was performed for 2 hours at 37°C in 10 µl reaction mixture containing: 10 nM RNA2 or 1 nM PP1, or RNA2/PP1 hybrid (final concentrations of RNA2 and PP1 were 10 nM and 1 nM, respectively), 75 ng yeast RNA, 2 µCi [α³³P] dATP, 1×Tango™, 1 mM dNTP in DEPC-treated water (Fermentas), with or without 1.5 U Phi29 DNA polymerase (all components, except [α³³P] dATP (Amersham Biosciences), yeast RNA (Pharmacia), were from Fermentas). After incubation 10 µl of 2×Loading dye solution (Fermentas) were added to stop the reaction. Samples were heated for 10 minutes at 70°C and placed on ice before loading onto acrylamide gel.

5 µl of samples were loaded onto 8% denaturing acrylamide gel and run on adjustable slab gel system (Cole-Parmer) at 250 V at room temperature for about 3 hours until bromophenol blue dye front exited the gel (gel parameters: length 20 cm, thickness 0.75 mm). Gel was dried and analyzed as described in Example 4, part A.

### B. Test of RCA efficiency (in the presence of RNaseV1)

PP1 was prepared as described in Example 4, part A.

RNA-DNA hybridization, RNA2 and PP1 preparation for cleavage reaction was performed as described in Example 10, part A.

Incubation with Phi29 DNA polymerase was performed as described in Example 10, part A, only reaction mixture contained 3·10⁻⁴ U RNaseV1 (Ambion). RNaseV1 was added the last, after mixing all other components on ice.

5 µl of samples were loaded onto 8% denaturing acrylamide gel, run and analyzed as described in Example 10, part A.

### Example 11

### A. Assay of Phi29 polymerase RNase activity on RNA1-PP1 hybrid

5'- end labeling of RNA1 was performed as described in Example 3, part A.

Padlock probe (PP1) was prepared as described in Example 4, part A.

Hydrolysis reaction was performed in 1xTango™ (Fermentas) buffer using 5'-end labeled RNA1 and PP1 in the molar ratio of 1/100 nM in the presence or absence of Phi29 DNA Polymerase.

First, hybridization reaction was carried out as described in Example 14, part A. After hybridization reaction mixture was supplemented with 3.75 U of Phi29 DNA polymerase. Reactions were carried out at 37°C for 15 min and terminated by adding equal volume of STOP Solution (Fermentas). Samples were incubated at 95°C for 10 min and left on ice.

Hydrolysis products were subjected to denaturing electrophoresis. The samples were loaded onto 10% acrylamide gel (29:1 acrylamide/bisacrylamide) with 7 M Urea and run at room temperature for 1.5 hours at 500 V in 1xTBE buffer (Fermentas). After electrophoresis, gel was dried and analyzed using Cyclone Storage Phosphor System and OptiQuant™ Image Analysis Software.

### B. Assay of Phi29 polymerase RNase activity on RNA2-PP1 hybrid

5-end labeling of RNA2 was performed as described in Example 7, part A.

Padlock probe (PP1) was prepared as described in Example 4, part A.

Hydrolysis reaction was performed in 1xTango™ (Fermentas) buffer using 5'-end labeled RNA2 and PP1 in the molar ratio of 1/100 nM in the presence or absence of Phi29 DNA polymerase.

First, hybridization reaction was carried out as described in Example 11, part A. After hybridization reaction mixture was supplemented with 3.75 U of Phi29 DNA polymerase. Reactions were carried out at 37°C for 15 min and terminated by adding an equal volume of 2x STOP Solution (Fermentas). Samples were incubated at 95°C for 10 min and left on ice.

Hydrolysis products were subjected to denaturing electrophoresis. The samples were loaded onto 10% acrylamide gel (29:1 acrylamide/bisacrylamide) with 7 M Urea and run at room temperature for 1.5 hours at 500 V in 1xTBE buffer (Fermentas). After electrophoresis, gel was dried and analyzed using Cyclone Storage Phosphor System and OptiQuant™ Image Analysis Software.

### Example 12

RNA padlock-probes hybridization effectiveness dependence on RNA length was studied using electrophoresis mobility shift assay (EMSA). Experiments were performed with 5'- labeled 48 nt and 111 nt RNA molecules (RNA1 and RNA2, respectively), where RNA1 constituted a portion of RNA2, and three different preformed padlock-probes (PP1, PP2, PP3).

### A. Gel shift analysis of RNA1 hybridization with different padlock probes

5'- end labeling of RNA1 was performed as described in Example 3, part A.

Padlock probe PP1 was prepared as described in Example 4, part A. PP2 and PP3 were prepared the same way as PP1 except that PP2 and PP3 oligonucleotides were circularized on PL2 and PL3 oligonucleotides, respectively.

Hybridization experiments were performed in 1xTango™ (Fermentas) buffer using 5'- end labeled RNA1 and each padlock probe separately in the following molar ratios: 1/1, 1/10, 1/100 nM. Control reaction was obtained by mixing 1xTango™ (Fermentas) buffer with 1 nM of 5'- end labeled RNA1. All incubations were carried out at 65°C for 10 min, then left for 10 min at room temperature and then placed on ice. Finally, glycerol was added to each sample to a final concentration of 15%.

Hybridization effectiveness was studied using EMSA. The samples were loaded onto 10% acrylamide gel (29:1 acrylamide/bisacrylamide) and run at room temperature for two hours at 200 V in 1×TBE buffer (Fermentas). After electrophoresis, gel was dried and analyzed using Cyclone Storage Phosphor System and OptiQuant™ Image Analysis Software and GrafPad Prism (version 4.03) program.

### B. Gel shift analysis of RNA2 hybridization with different padlock probes

5-end labeling of RNA2 was performed as described in Example 7, part A.

Padlock probe PP1 was prepared as described in Example 4, part A. PP2 and PP3 were prepared the same way as PP 1 except that PP2 and PP3 oligonucleotides were circularized on PL2 and PL3 oligonucleotides, respectively.

Hybridization experiments were performed in 1xTango™ (Fermentas) buffer using 5'- end labeled RNA2 and each padlock probe separately in the following molar ratios: 1/1, 1/10, 1/100 nM. Control reaction was obtained by mixing 1xTango™ (Fermentas) buffer with 1 nM of 5'- end labeled RNA2. All incubations were carried out at 65°C for 10 min, then left for 10 min at room temperature and then placed on ice. Finally, glycerol was added to each sample to a final concentration of 15%.

Hybridization effectiveness was studied using EMSA. The samples were loaded onto 10% acrylamide gel (29:1 acrylamide/bisacrylamide) and run at room temperature for two hours at 200 V in 1xTBE buffer (Fermentas). After electrophoresis, gel was dried and analyzed using Cyclone Storage Phosphor System and OptiQuant™ Image Analysis Software and and GrafPad Prism (version 4.03) program.

### Example 13

### A. Gel shift analysis of RecA promotion of RNA hybridization with circularized padlock probes

5-end labeling of RNA2 was performed as described in Example 7, part A.

Padlock probe (PP2) was prepared as described in detail in Example 4, part A, except that PP2 oligonucleotide was circularized on PL2 oligonucleotide.

Circularized single stranded DNA was first preincubated with RecA protein at 37°C for 30 min. Reaction mixtures with 1 or 10 nM PP2 were prepared in 33 mM HEPES-KOH buffer pH 7.5 containing 3 mM ATP and 1 mM Mg acetate. The ratio of RecA monomers to nucleotide residues of single stranded DNA was 1/2, respectively.

Preincubation reaction was followed by addition of 5'- end labeled RNA2 to a final concentration of 1 nM and of Mg acetate bringing its concentration to 12 mM. Hybridization was performed at 37°C for 30 min. Control reaction, where RecA and PP2 were omitted, was performed at the same conditions.

All reactions were terminated by the combined treatment with proteinase K (Fermentas) and SDS, at final concentrations of 200 µg/ml and 0.4%, respectively, in 37°C for 30 min. Finally, glycerol was added to each sample to a final concentration of 15% and hybridization effectiveness was studied using electrophoresis mobility shift assay (EMSA) as described in Example 11, part A.

### B. Gel shift analysis of RecA promotion of RNA hybridization with padlock probe oligonucleotides

5-end labeling of RNA2 was performed as described in Example 7, part A.

Single stranded DNA (PP2 oligonucleotide) was first preincubated with RecA protein at 37°C for 30 min. Reaction mixtures with 1 or 10 PP2 oligonucleotide were prepared in 33 mM HEPES-KOH buffer pH 7.5 containing 3 mM ATP and 1 mM Mg acetate. The ratio of RecA monomers to nucleotide residues of single stranded DNA was 1/2, respectively.

Preincubation reaction was followed by addition of 5'- end labeled RNA2 to a final concentration of 1 nM and of Mg acetate bringing its concentration to 12 mM. Hybridization was performed at 37°C for 30 min. Control reaction, where RecA and PP2 were omitted, was performed at the same conditions.

All reactions were terminated by a combined treatment with proteinase K (Fermentas) and SDS, at final concentrations of 200 µg/ml and 0.4%, respectively, in 37°C for 30 min. Finally, glycerol was added to each sample to a final concentration of 15% and hybridization effectiveness was studied using electrophoresis mobility shift assay (EMSA) as described in Example 11, part A.

### Example 14 (Figure 14)

To test whether Phi29 DNA polymerase may be used for detection of the target RNA in the mix of different RNA fragments, the experiments were carried out with LR RNA ladder and preformed padlock probe (PP2).

### A. PP2 incubation with Phi29 DNA polymerase

PP2 was prepared as described in Example 4, part A, only PP2 oligonucleotide was circularized on PL2 oligonucleotide.

PP2 for incubation with Phi29 DNA polymerase reaction was prepared by diluting PP2 to 20 nM in 225 µl of hybridization mixture with 1×hybridization buffer (50 mM Tris-HCl pH 8.0, 0.1 mM EDTA, 80 mM NaCl) in DEPC-treated water (Fermentas), heated for 10 minutes at 65°C, cooled to room temperature for 10 minutes and placed on ice.

Incubation with Phi29 DNA polymerase was performed for 2 hours at 37°C in 50 µl reaction mixture containing: 10 nM PP2, 1 mM DTT, 1×Tango™, 1 mM dNTP, 1 U/µl Ribolock™ ribonuclease inhibitor in DEPC-treated water with or without 7.5 U Phi29 DNA polymerase (Fermentas). After incubation 10 µl of 6×Orange loading dye solution (15% Ficoll® 400, 10 mM Tris-HCl pH 7.5, 100 mM EDTA, 1% SDS, 0.15% Orange G) were added to stop the reaction, samples were heated for 10 minutes at 65°C and placed on ice before loading onto agarose gel.

12 µl of samples were loaded onto 1% agarose gel [1% agarose, 0.4 mg/ml ethidium bromide, 1×TAE (Fermentas)] and run at 5 V/cm until the Orange G dye migrated 2/3 of the gel length. Gel was visualized and photographed under Ultra Lum Electronic U.V. Transilluminator (Ultra-Lum).

### B. RNA LR Ladder incubation with Phi29 DNA polymerase

RNA LR Ladder (Fermentas) for incubation with Phi29 DNA polymerase reaction was prepared as PP2 as described in Example 14, part A, only 5 µg of RNA LR Ladder (containing target transcript, which was one of the RNA size standards) were added to 225 µl of sample.

RNA LR Ladder incubation with Phi29 DNA polymerase was performed as described Example 15, part A, only the substrate was 0.56 µg of RNA LR Ladder in 50 µl of reaction mixture.

12 µl of samples were loaded onto 1% agarose gel, run and photographed as described in Example 15, part A.

### C. Generation of RCA product with Phi29 DNA polymerase from RNA LR Ladder, hybridized with PP2

PP2 was prepared as described in Example 4, part A, only PP2 oligonucleotide was circularized on PL2 oligonucleotide.

RNA LR Ladder and PP2 hybridization was performed in 225 µl sample, containing 5 µg of RNA LR Ladder and 20 nM PP2 respectively, under the same conditions as PP2 preparation for incubation with Phi29 DNA polymerase as described in Example 14, part A.

RNA LR Ladder-PP2 hybrid incubation with Phi29 DNA polymerase was performed as described in Example 14, part A, only the substrate was RNA LR Ladder-PP2 hybrid at 0.56 µg RNA LR Ladder and 10 nM PP2, respectively.

12 µl of samples were loaded onto 1% agarose gel, run and photographed as described in Example 14, part A.

### D. Incubation of RNA LR Ladder mixed with PP4 with Phi29. DNA polymerase

PP4 was prepared as described in Example 4, part A, only PP4 oligonucleotide was circularized on PL4 oligonucleotide.

RNA LR Ladder and PP4 were mixed and prepared for incubation with Phi29 DNA polymerase in the sample, containing 5 µg RNA LR Ladder and 20 nM PP4 respectively. Sample was heated and cooled under the same conditions as PP4 preparation for incubation with Phi29 DNA polymerase as described in Example 14, part A.

RNA LR Ladder and PP4 mixture incubation with Phi29 DNA polymerase was performed as described in Example 14, part A, only the substrate was 0.56 µg RNA LR Ladder and 10 nM PP4 mixture in 50 µl.

12 µl of samples were loaded onto 1% agarose gel, run and photographed as described in Example 14, part A.

### Example 15 (Figure 15)

### A. Generation of RCA product with Phi29 DNA polymerase from the mix of RNA transcripts, hybridized with PP, in the absence of RNases.

PP 1 and PP4 were prepared as PP1 described in Example 4, part A, only PP4 oligonucleotide was circularized on PL4 oligonucleotide.

For RNA-DNA hybridization RNA LR Ladder (containing target transcript, which was one of the RNA size standards) (Fermentas) and PP1 or PP4 were diluted to a final concentration of 0.36 µg RNA LR Ladder and 4 nM PP, respectively, in 40 µl of hybridization mixture with 1×Tango™ buffer in DEPC-treated water (Fermentas). Samples were heated for 10 minutes at 65°C, cooled to room temperature for 10 minutes and placed on ice.

RNA LR Ladder for cleavage reaction was prepared by diluting 0.36 µg RNA LR Ladder in 40 µl with 1×Tango™ buffer in DEPC-treated water (Fermentas), heating and cooling as described above. PP1 and PP4 for cleavage reaction were prepared by diluting PP1 or PP4 to 4 nM in 40 µl with 1×Tango™ buffer in DEPC-treated water (Fermentas), heating and cooling as described above.

Incubations with Phi29 DNA polymerase were performed for 2 hours at 37°C in 20 µl reaction mixture containing: RNA LR Ladder and PP at 45 ng and 1 nM concentrations, respectively, (or 45 ng RNA LR Ladder, or 1 nM PP1, or 1 nM PP4), 150 ng yeast RNA (Pharmacia), 1 mM dNTP, 1×Tango™, with or without 3 U Phi29 DNA polymerase in DEPC-treated water (Fermentas). After incubation 4 µl of 6×Orange loading dye solution (Fermentas) were added to stop the reaction, samples were heated for 10 minutes at 65°C and placed on ice before loading onto agarose gel.

12 µl of samples were loaded onto 1% agarose gel, run and photographed as described in Example 14, part A.

### B. Generation of RCA product with Phi29 DNA polymerase in the presence of RNaseIII from the mix of RNA transcripts, hybridized with PP

All manipulations were performed exactly as described in Example 15, part A, except for RCA reaction mixture. Incubations with Phi29 DNA polymerase were performed as described in Example 15, part A, only 16·10⁻³ U of RNaseIII (Epicentre) were added to the reaction mixtures. RNase III was added the last, after mixing of all other components on ice.

### C. Generation of RCA product with Phi29 DNA polymerase in the presence of RNase V1 from the mix ofRNA transcripts, hybridized with PP

All manipulations were performed exactly as described in Example 15, part A, except for RCA reaction mixture. Incubations with Phi29 DNA polymerase were performed as described in Example 15, part A, only 10⁻⁴ U of RNase V1 (Ambion) were added to the reaction mixtures. RNase III was added the last, after mixing of all other components on ice.

### Example 16 (Figure 16)

### A.GAPDH gene transcript detection using ligation-based RCA

GAPDH gene transcript was detected by performing ligation of target-specific DNA on RNA substrate, followed by RNA cleavage and RCA with Phi29 DNA polymerase. RCA product was labeled during RCA reaction by incorporating labeled nucleotide and tested by hybridization with specific oligonucleotide, followed by restriction of formed hybrid with Mva1269I restriction endonuclease.

GAPDH gene transcript was synthesized using T7 RNA polymerase from linerised plasmid pTZ A1K4 (provided by Nuclear Standard Laboratory, Fermentas), containing GAPDH gene insert. After transcription GAPDH gene transcript was treated with DNaseI (Fermentas) under standard conditions followed by multiple precipitation using NH₄CH₃COO and isopropanol. Final RNA concentration was estimated spectrophotometricaly.

Target-specific DNA ligation on RNA was performed by hybridization and ligation of phosphorylated circular PP9 oligonucleotide on target RNA. PP9 oligonucleotide was phosphorylated as PP1 as described in Example 4, part A. RNA/DNA hybridization was performed in 10 µl of reaction mixture, containing: 1 × Tango™ buffer (Fermentas), 5nM GAPDH transcript, 50 nM phosphorylated PP9 or PP10. Hybridization samples were heated at 65°C for 3 minutes, cooled to room temperature for 10 minutes and placed on ice. Control reactions, containing only 5nM of GAPDH transcript in 1 × Tango™ buffer or only 50 nM of phosphorylated PP9 in 1 × Tango™ buffer experienced the same hybridization conditions.

Ligation reaction was performed for 2 hours at 37°C, after adding ATP to the final concentration of 10 µM and 3.75 U/µl T4 DNA ligase to the hybridization samples. Ligation step was followed by ligase inactivation at 70°C for 10 min, sample cooling to room temperature for 10 min. and placing on ice.

RCA reactions were performed by adding 4 µl of RCA components to ligation reaction samples {at a final concentrations in 15 µl sample: 80 nM [α³³P]dATP (Amersham Biosciences), 0.1 mM dNTP, 1 × Tango™ buffer, 2.25 U phi29 DNA polymerase in DEPC-treated water}. RCA samples were incubated for 3 hours at 37°C, heated for 10 min at 70°C and placed on ice.

5 µl of RCA product were tested for product specificity. Therefore, it was hybridized with specific oligonucleotide (containg Mva1269I restriction endonuclease site) at a final concentration of 1 µM in 2 × Tango™ buffer in 7 µl of sample using slow cooling program in PCR cycler {95°C 5 min., 90°C 2 min., 80°C 2 min., 70°C 2 min, 60°C 2 min., 50°C 2 min., 40°C 2 min., 30°C 2 min., 20°C 2 min., 10°C 2 min., on ice}. Cleavage step was performed for 1 hour at 37°C in the presence or absence of 5 U of restriction endonuclease Mva1269I (10 U/µl, Fermentas). Mva1269I was inactivated by heating reaction sample at 70°C for 10 minutes.

Finally, samples were treated with SDS and proteinase K [at a final concentrations of 0.4% SDS and 200 µg/ml proteinase K] for 30 min at 37°C. Following incubation, the equal volume of STOP solution (Fermentas) was added to the samples, which were denatured for 5 min. at 95°C and placed on ice.

5 µl of samples were loaded onto 8% denaturing acrylamide gel and run at 600 V as described in Example 3, part B. Gel was dried and analyzed as described in Example 3, part A.

### B. GAPDH gene transcript detection using ligation-based RCA in the presence of RNase III

All steps were the same as described in Example 16, part A except for hybridization step. RNA/DNA hybridization was performed in 9 µl of reaction mixture, containing: 1 × Tango™ buffer (Fermentas), 5nM GAPDH transcript, 50 nM phosphorylated PP9 or PP10. Hybridization samples were heated at 65°C for 3 min, cooled to room temperature for 10 min and followed by adding 1U of RNaseIII (Epicentre). Samples were incubated at 37°C for 30 min followed by inactivation of RNaseIII at 70°C for 10 min and once more cooled to room temperature for 10 min. Control reactions, containing only 5 nM of GAPDH transcript in 1 × Tango™ buffer or only 50 nM of phosphorylated PP9 in 1 × Tango™ buffer experienced the same hybridization conditions including addition of RNaseIII.

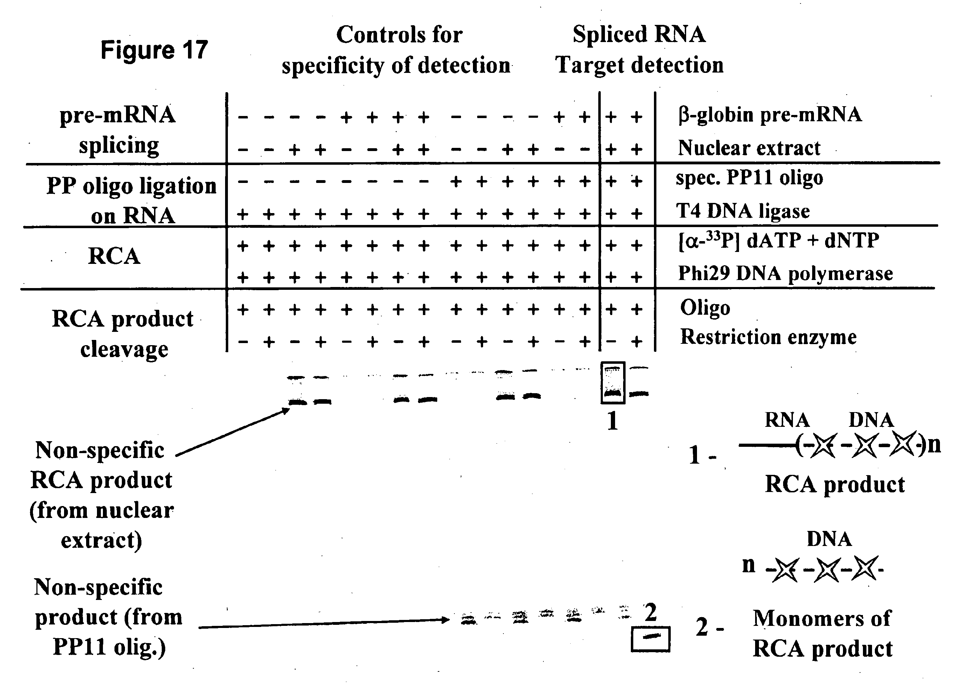

### Example 17 (Figure 17) spliced β-globin transcript

### Spliced mRNA target detection using ligation-based RCA

Rabbit β-globin pre-mRNA was spliced *in vitro* and the spliced mRNA variant was detected by performing ligation of target-specific DNA on RNA substrate, followed by RNA cleavage and RCA with Phi29 DNA polymerase. RCA product was labeled during RCA reaction by incorporating labeled nucleotide and tested by hybridization with specific oligonucleotide, followed by restriction of formed hybrid with RsaI restriction endonuclease.

Rabbit β-globin pre-mRNA was synthesized with T7 RNA polymerase from plasmid-generated PCR fragment with incorporated T7 promoter.

PCR fragment was generated from recombinant plasmid pSP64-R chr β-globin (a gift from dr. A. Kanopka; Institute of Biotechnology, Lithuania), containing rabbit β-globin gene as the insert. PCR reaction was performed in 300 µl mixture, containing: 1 × Taq buffer with (NH₄)SO₄, 1.5 mM MgCl₂, 0.2 mM dNTP, 15 ng template DNA, 1 µM direct primer with T7 promoter (5'- ATT AAT ACG ACT CAC TAT AGA ATA CAA GCT TGG GCT G-3'), 1 µM reverse primer (5'- GAG GAC AGG TCC CCA AAG-3'), 7.5 U recombinant Taq DNA polymerase in nuclease-free water (all PCR reagents were from Fermentas). Reaction cycles were as follows: 2 minutes at 95°C, {1 minute at 95°C, 1 minute at 49°C, 2 minutes at 72°C - repeated 24 times}, 10 minutes at 72°C and cooled down. Generated β-globin PCR fragment was loaded on low melting point 1 % TopVision™ LM GQ agarose gel and recovered with agarase by using standart agarase protocol (Fermentas).

β-globin pre-mRNA synthesis was performed for 2 hours at 37°C using T7 transcription kit (Fermentas) in a 50 µl mixture containing: 0.5 mM ATP, 0.5 mM CTP, 0.5 mM UTP, 0.05 mM GTP, 0.5 mM m⁷G(5')ppp(5')G (Amersham Biosciences), 1 × transcription buffer, 50 U Ribolock™ Inhibitor, 1 µg β-globin PCR fragment and 40 U T7 RNA polymerase in DEPC-treated water. The transcript was treated with 2U DNaseI (Ambion) for 30 minutes at 37°C and purified on Microspin™ G-25 column (Amersham Biosciences), phenol/chloroform extracted, precipitated with 1/10 volume of sodium acetate (3 M, pH5.2), 0.05 µg/µl glycogen, 2.5 volume of ethanol at -70°C overnight. Sample was centrifuged at +4°C for 15 minutes at 25000 × g, pellet was washed with 0.5 ml ethanol (70 %), centrifuged at +4°C for 10 minutes at 25000 × g, dried at room temperature for several minutes and diluted in 10 µl of DEPC-treated water.

β-globin pre-mRNA splicing was performed for 2 hours at 30°C in 25 µl of reaction mixture, cointaining: 2.6% polyvinyl alcohol (Sigma), 3.2 mM MgCl₂, 20 mM creatinphosphate (Sigma), 2 mM ATP, 25 U Ribolock™ Inhibitor (Fermentas), with or without 30.2 nM β-globin pre-mRNA, 10 µl nuclear extract (a gift from Dr. A. Kanopka; Institute of Biotechnology, Lithuania). The sample was adjusted to 25 µl with buffer D [20 mM HEPES, pH 7.9, 20% glycerol, 100 mM KCl, 0.2 mM EDTA, 0.5 mM DTT]. The last components added to reactions were β-globin pre-mRNA and nuclear extract. After the incubation, samples were diluted to 100 µl with DEPC-treated water, following the addition of 100 µl proteinase K buffer [0.02 M Tris-HCl pH 7.8, 0.01 M EDTA, 1 % SDS], 2 mg yeast RNA (Pharmacia), 36 mg proteinase K (Fermentas) and mixtures were incubated for 30 minutes at 37°C, phenol/chloroform extracted and precipitated with ethanol as described above.

Target-specific DNA ligation on RNA was performed by hybridization and ligation of phosphorylated circular PP11 oligonucleotide on target RNA. PP11 oligonucleotide was phosphorylated as PP1 as described in Example 4, part A. RNA/DNA hybridization was performed in 10 µl of reaction mixture, containing: 1 × Tango™ buffer (Fermentas), 0.66 µl of each splicing reaction sample, with or without 50 nM phosphorylated PP11 in DEPC-treated water. Hybridization samples were heated at 65°C for 3 minutes, cooled to room temperature for 10 minutes, incubated at 37°C for 30 minutes and placed on ice. 2 µl of ligation reaction components were added to the hybridization samples [at a final concentrations in 12 µl sample: 10 µM ATP, 1 × Tango™ buffer, 0.5 U/µl T4 DNA ligase in DEPC-treated water] and the reactions were incubated for 2 hours at 37°C, heated for 10 min. at 70°C, cooled to room temperature for 10 min. and placed on ice.

RCA reactions were performed by adding 3 µl of RCA components to ligation reaction samples [at a final concentrations in 15 µl sample: 80 nM [α³³P]dATP (Amersham Biosciences), 0.1 mM dNTP, 1 × Tango™ buffer, 2.25 U phi29 DNA polymerase in DEPC-treated water]. RCA samples were incubated for 3 hours at 37°C, heated for 10 min. at 70°C and placed on ice.

5 µl of RCA product were tested by hybridization with specific oligonucleotide (containing RsaI restriction endonuclease) site at a final concentration of 1 µM in 1 × Tango™ buffer in 6 µl of sample using slow cooling program in PCR cycler {95°C 5 min., 90°C 2 min., 80°C 2 min., 70°C 2 min, 60°C 2 min., 50°C 2 min., 40°C 2 min., 30°C 2 min., 20°C 2 min., 10°C 2 min., on ice}. The formed hybrid was cleaved with 5 U of restriction endonuclease RsaI (10 U/µl, Fermentas) for 1 hour at 37°C and heated for 10 minutes at 70°C. For the control reactions 0.5 µl of water was used instead of RsaI.

After the restriction reaction, samples were treated with SDS and proteinase K [at a final concentrations of 0.4 % SDS and 200 µg/ml proteinase K] for 30 min. at 37°C. Following incubation, the equal volume of STOP solution (Fermentas) was added to the samples, which were denatured for 5 min. at 95°C and placed on ice.

5 µl of samples were loaded onto 8% denaturing acrylamide gel and run at 600 V as described in Example 3, part B. Gel was dried and analyzed as described in Example 3, part A.

### Example 18 (Figure 18)

### Ligation-based RNA target detection in RNA transcripts mix by RCA

RNA target detection was done by performing ligation of target-specific DNA on RNA substrate, followed by RNA cleavage and RCA with Phi29 DNA polymerase.

PP2 and PP4 oligonucleotides were 5'-phosphorylated at a final concentration of 10 µM in 10 µl of reaction mixture [1×Tango™, 50 µM ATP, 5 U T4 PNK in DEPC-treated water (all components were from Fermentas)] for 30 minutes at 37°C. Following the incubation reaction mixture was heated for 10 minutes at 70°C and placed on ice. 2 µl of phosphorylated PP2 or PP4 oligonucleotide were added to hybridization and ligation on RNA LR Ladder or specific DNA targets.

RNA-DNA hybridization and ligation reactions were performed in 50 µl of reaction mixture, containing either 400 nM phosphorylated PP oligonucleotide or 0.89 µg RNA LR Ladder (one of the RNA size standards was target transcript), or both of them, 1×ligation buffer (10 mM Tris-HCl pH 7.5, 10 mM MgCl₂, 20 µM ATP) in DEPC-treated water and 1 U/µl Ribolock™ ribonuclease inhibitor and/without 0.5 U/µl T4 DNA ligase, which were added after hybridization step. Samples were heated for 3 minutes at 65°C, cooled to room temperature for 10 minutes and placed on ice. Ribolock™ ribonuclease inhibitor with or without T4 DNA ligase were added and reaction mixtures were incubated for 2 hours at 37°C following 10 minutes incubation at 70°C and placed on ice.

DNA-DNA hybridization and ligation reactions were performed exactly in the same way, only the substrates were hybrids of oligonucleotides: 400 nM phosphorylated PP2 oligonucleotide and 80 nM PL2 oligonucleotide and 400 nM phosphorylated PP4 oligonucleotide and 80 nM PL4 oligonucleotide, respectively.

12.5 µl of ligation probes were added to the incubation with Phi29 DNA polymerase reaction mixture, containing 1×Tango™ buffer, 1 mM DTT, 1 mM dNTP, 1 U/µl Ribolock™ ribonuclease inhibitor in DEPC-treated water with or without 7.5 U Phi29 DNA polymerase (all components were from Fermentas) for the final reaction volume, that was adjusted to 50 µl. Samples were incubated for 2 hours at 37°C, following the addition of 10 µl of 6×Orange loading dye solution. Samples were heated for 10 minutes at 65°C and placed on ice before loading onto agarose gel.

12 µl of samples were loaded onto 1% agarose gel, run and photographed as described in Example 14, part A.

### Example 19 (Example 19)

### Ligation-based GAPDHgene transcript detection in mRNA mixture by RCA

PP5 and PP6 oligonucleotides were phosphorylated as described in Example 4, part A.

Hybridization reaction was performed at 65°C for 5 min (then at room temperature for 10 min) in 9 µl volume of 1xtango™ (Fermentas), containing 10 µCi [α³³-P], 0.1 mM ATP, 1 mM dNTP mix, 0.25 µg mRNA (Ambion) and 0.1 nM PP5 or PP6 oligonucleotides. The latter oligonucleotides were absent in control reactions. Further, 0.25 µl (40 U/µl) of Ribolock™ (Fermentas) and 0.5 µl (5 U/µl) of T4 DNA Ligase (Fermentas) were added to each sample and incubation at 37°C for 2 hours was performed. Ligase was heat inactivated at 70°C for 10 min. RCA reaction, initiated by adding of 0.15 U of Phi29 DNA polymerase into 10 µl of each sample, lasted for 3 hours at 37°C and was terminated at 70°C for 10 min.

Further, RCA product specificity was tested. Complementary oligonucleotides, containing Mva1269I restriction endonuclease recognition site, was annealed to RCA product in 2xTango™ buffer (Fermentas) in 10 µl volume at 10 µM final concentration. Annealing step was performed in MasterCycler (Eppendorf): 95°C for 5 min, 95°C for 2 min, 95°C for 2 min, 80°C for 2 min, 70°C for 2 min, 60°C for 2 min, 50°C for 2 min, 40°C for 2 min, 30°C for 2 min, 20°C for 2 min, 10°C for 2 min. Then each sample was divided into two portions and one of them was supplemented with 5 U of Mva1269I restriction endonuclease. Monomerization reaction was performed at 37°C for an hour.

Before electrophoretic analysis, all samples were treated with proteinase K (Fermentas) and SDS, at final concentrations of 200 µg/ml and 0.4%, respectively, in 37°C for 30 min. Then equal volume of 2x STOP Solution (Fermentas) was added and incubation at 95°C for 10 min was performed.

Samples were analyzed under denaturing conditions as described in Example 13, part B.

### Example 20 (Figure 20)

### Improvement of GAPDH gene transcript RCA detection by RecA addition in mRNA mixtures

All manipulations were performed exactly as described in Example 19, except that hybridization and ligation steps were performed as described in Example 13, part B.

### Example 21(Figure 21)

### A. GAPDH gene transcript detection in situ by RCA using PP5 in HeLa cell line

40 µl of the HeLa cell line were cultured in 10 well glass slide (Electron Microscopy Sciences) in Dulbecco's Modified Eagles medium without L-glutamine (Sigma), supplemented with 10% fetal bovine serum (Biochrom AG), 300 µg/ml L-glutamine, 50 µg/ml gentamycine sulphate (Sigma) for 24 hours at 37°C in CO₂ incubator.

The cells on the slide were washed with 1×PBS buffer (137 mM NaCl, 2.7 mM KCl, 100 mM Na₂HPO₄, 2 mM KH₂PO₄, pH 7.4) 2 times for 5 minutes, following dehydration and fixation by immersing slides into 70%, 85%, 96% ethanol for 3 minutes each. After dehydration, glass slides were dried at room temperature for 2 minutes.

PP5 oligonucleotide was phosphorylated as PP1 oligonucleotide as described in Example 4, part A.

GAPDH transcript and PP5 oligonucleotide hybridization reaction was performed on dehydrated cells in the well of a glass slide in 30 µl of 1×hybridization buffer [20% formamide, 2xSSC (300 mM NaCl, 30 mM sodium citrate, pH 7.0), 5% glycerol], containing 100 nM PP5 for 1 hour at 37°C. Glass slide was washed at 37°C in pre-heated washing buffer (0.1 M Tris pH 7.5, 0.15 M NaCl, 0.05% Tween 20) 2 times for 5 minutes, dehydrated and dried as described above.

The ligation of PP5 on GAPDH transcripts was performed in 30 µl of 1×T4 DNA ligase buffer, containing 0.2 µg/µl BSA, 4 U/µl Ribolock™ Ribonuclease inhibitor, 0.1 U/µl T4 DNA ligase (all components were from Fermentas) for 30 minutes at 37°C in a glass slide well. Glass slide was washed at 37°C in pre-heated washing buffer one time for 3 minutes; dehydrated and dried as described above.

RCA was performed in 30 µl of 1×Phi29 DNA polymerase buffer, containing 0.2 µg/µl BSA, 250 µM dNTP, 4 U/µl Ribolock™ Ribonuclease inhibitor, 1 U/µl Phi29 DNA polymerase (all components were from Fermentas) for 1 hour at 37°C. Glass slide was washed, dehydrated and dried as described in the ligation step.

RCA product was detected by hybridization with DP-FITC oligonucleotide in 30 µl of 1×hybridization buffer, containing 2 nM DP-FITC oligonucleotide, for 45 minutes at 37°C. Glass slide was washed, dehydrated and dried as described in the hybridization step above.

The glass slide well, containing fixated cells, was treated with 10 µl of mounting solution (1 mg/ml ρ-phenylenediamine, 100 ng/ml DAPI, 90% glycerol in 1×PBS, pH 8.0 (adjusted with sodium bicarbonate) and covered with cover slip. The slide was visualized and photographed using fluorescence microscope (Olympus IX70), equipped with a 100W mercury lamp, a CCD camera (CoolSNAP-Pro Monochrome Camera, Media Cybernetics) and the excitation and emission filters for visualization of FITC, DAPI. A 100x immersion objective was used for image generation and image was captured using 4 s (FITC) or 100 ms (DAPI) exposure time. Image was collected using the Image-Pro Plus software (Media Cybernetics).

### B. GAPDHgene transcript detection in situ by RCA using PP6 in HeLa cell line

HeLa cell line was cultured and cells were washed as described in Example 21, part A.

PP6 oligonucleotide was phosphorylated as PP1 oligonucleotide as described in Example 4, part A.

GAPDH transcript and PP6 oligonucleotide hybridization, ligation reactions and RCA were performed as described in Example 21, part A, only PP6 oligonucleotide was used instead of PP5 oligonucleotide.

RCA product was detected, visualized and photographed as described in Example 21, part A.

### Example 22 (Figure 22)

### A. Beta-actin gene transcript detection by RCA in situ using PP7 and RecA protein in HeLa cell line

HeLa cell line was cultured and cells were washed as described in Example 21, part A. PP7 oligonucleotide was phosphorylated as PP1 oligonucleotide as described in Example 4, part A.

200 nM PP7 oligonucleotide was incubated with 4.3µM RecA protein in 15µl 2x SSC buffer for 40 minutes at 37°C. Cells on the glass slide were fixed and dehydrated as described in Example 19, part A.

15µl of PP7 oligonucleotide and RecA mixture were hybridized with beta-actin transcript in the 15µl of 1×hybridization buffer for 1 hour at 37°C.

Ligation reaction was performed as described in Example 21, part A, only PP7 oligonucleotide was used instead of PP5 oligonucleotide. Glass slide was washed at 37°C in pre-heated washing buffer 2 times for 5 minutes, dehydrated and dried as described in Example 19, part A.

RCA was performed as described in Example 21, part A, only PP7 oligonucleotide was used instead of PP5 oligonucleotide. RCA product was detected, visualized and photographed as described in Example 21, part A.

### B. Beta-actin gene transcript detection by RCA in situ using PP8 and RecA protein in HeLa cell line

HeLa cell line was cultured and cells were washed as described in Example 21, part A. PP8 oligonucleotide was phosphorylated as PP1 oligonucleotide as described in Example 4, part A.

Incubation of PP8 oligonucleotide and RecA protein was performed as described in Example 20, part A. PP8 oligonucleotide and RecA protein mixture were hybridized with beta-actin transcript as described in Example 22, part A only PP8 oligonucleotide was used instead of PP7 oligonucleotide.

Ligation reaction was performed as described in Example 21, part A, only PP8 oligonucleotide was used instead of PP5 oligonucleotide. Glass slide was washed as described in Example 22, part A, dehydrated and dried as described in Example 21, part A.

RCA was performed as described in Example 21, part A, only PP8 oligonucleotide was used instead of PP5 oligonucleotide. RCA product was detected, visualized and photographed as described in Example 21, part A.

### C. Beta-actin gene transcript detection by RCA in situ using PP7 without RecA protein in HeLa cell line

HeLa cell line was cultured and cells were washed as described in Example 19, part A. PP7 oligonucleotide was phosphorylated as PP1 oligonucleotide as described in Example 4, part A.

200 nM PP7 oligonucleotide was incubated in 15µl 2x SSC buffer for 40 minutes at 37°C. PP7 oligonucleotide hybridization with beta-actin transcript was performed as described in Example 22, part A.

Ligation reaction was performed as described in Example 21, part A, only PP7 oligonucleotide was used instead of PP5 oligonucleotide. Glass slide was washed as described in Example 22, part A, dehydrated and dried as described in Example 21, part A.

RCA was performed as described in Example 21, part A, only PP7 oligonucleotide was used instead of PP5 oligonucleotide. RCA product was detected, visualized and photographed as described in Example 21, part A.

### D. Beta-actin gene transcript detection by RCA in situ using PP8 without RecA protein in HeLa cell line

HeLa cell line was cultured and cells were washed as described in Example 21, part A. PP8 oligonucleotide was phosphorylated as PP1 oligonucleotide as described in Example 4, part A.

200 nM PP8 oligonucleotide was incubated in 15 µl 2x SSC buffer for 40 minutes at 37°C. PP8 oligonucleotide hybridization with beta-actin transcript was performed as described in Example 22, part A, only PP8 oligonucleotide was used instead of PP7 oligonucleotide.

Ligation reaction was performed as described in Example 21, part A, only PP8 oligonucleotide was used instead of PP5 oligonucleotide. Glass slide was washed as described in Example 20, part A, dehydrated and dried as described in Example 21, part A.

RCA was performed as described in Example 21, part A, only PP8 oligonucleotide was used instead of PP5 oligonucleotide. RCA product was detected, visualized and photographed as described in Example 21, part A.

### Example 23 (Figure 23)

### A. Beta-actin gene transcript detection by RCA in situ using PP12, RecA protein and RNaseIII in HeLa cell line

HeLa cell line was cultured and cells were washed as described in Example 21, part A. PP12 oligonucleotide was phosphorylated as PP1 oligonucleotide as described in Example 4, part A.

Incubation of PP12 oligonucleotide and RecA protein was performed as described in Example 22, part A. PP12 oligonucleotide and RecA protein mixture was hybridized with beta-actin transcript in the 15 µl of 1×hybridization buffer (buffer composition described in Example 21, part A) with 10mM Mg acetate and 0.003 U/µl RNase III (Epicentre) for 1 hour at 37°C.

Ligation reaction was performed as described in Example 21, part A, only PP12 oligonucleotide was used instead of PP5 oligonucleotide. Glass slide was washed at 37°C in pre-heated washing buffer 2 times for 5 minutes, dehydrated and dried as described in Example 21, part A.

RCA was performed as described in Example 21, part A, only PP12 oligonucleotide was used instead of PP5 oligonucleotide. RCA product was detected, visualized and photographed as described in Example 21, part A.

### B. Beta-actin gene transcript detection by RCA in situ using PP13, RecA protein and RNaseIII in HeLa cell line

HeLa cell line was cultured and cells were washed as described in Example 21, part A. PP13 oligonucleotide was phosphorylated as PP1 oligonucleotide as described in Example 4, part A.

Incubation of PP13 oligonucleotide and RecA protein was performed as described in Example 22, part A. Hybridization reaction was performed as described in Example 23, part A, only PP13 oligonucleotide was used instead of PP12 oligonucleotide.

Ligation reaction was performed as described in Example 21, part A, only PP13 oligonucleotide was used instead of PP5 oligonucleotide. Glass slide was washed as described in Example 22, part A, dehydrated and dried as described in Example 21, part A.

RCA was performed as described in Example 21, part A, only PP13 oligonucleotide was used instead of PP5 oligonucleotide. RCA product was detected, visualized and photographed as described in Example 21, part A.

### C. Beta-actin gene transcript detection by RCA in situ using PP12, RecA protein, without RNaseIII in HeLa cell line

HeLa cell line was cultured and cells were washed as described in Example 21, part A. PP12 oligonucleotide was phosphorylated as PP1 oligonucleotide as described in Example 4, part A.

Incubation of PP12 oligonucleotide with RecA protein and hybridization reaction were performed as described in Example 22, part A, only PP12 oligonucleotide was used instead of PP7 oligonucleotide.

Ligation reaction was performed as described in Example 21, part A, only PP12 oligonucleotide was used instead of PP5 oligonucleotide. Glass slide was washed as described in Example 22, part A, dehydrated and dried as described in Example 21, part A.

RCA was performed as described in Example 21, part A, only PP12 oligonucleotide was used instead of PP5 oligonucleotide. RCA product was detected, visualized and photographed as described in Example 21, part A.

### D. Beta-actin gene transcript detection by RCA in situ using PP13, RecA protein, without RNaseIII in HeLa cell line

HeLa cell line was cultured and cells were washed as described in Example 21, part A. PP13 oligonucleotide was phosphorylated as PP1 oligonucleotide as described in Example 4, part A.

Incubation of PP13 oligonucleotide with RecA protein and hybridization reaction were performed as described in Example 22, part A, only PP13 oligonucleotide was used instead of PP7 oligonucleotide.

Ligation reaction was performed as described in Example 19, part A, only PP13 oligonucleotide was used instead of PP5 oligonucleotide. Glass slide was washed as described in Example 22, part A, dehydrated and dried as described in Example 21, part A.

RCA was performed as described in Example 21, part A, only PP13 oligonucleotide was used instead of PP5 oligonucleotide. RCA product was detected, visualized and photographed as described in Example 21, part A.

### RNA and DNA sequences:

SEQ ID No: 1
   **RNA1 oligonucleotide:**
      CGGGAUACCGUCCAGCGACAUUCUUCCUCGGUACAUAAUCUCCUUUGG
SEQ ID No: 2
   **RNA2 oligonucleotide:** 
5'-
GGGAAAGCUUUACAUUUUCGCGAUACCGUCCAGCGACAUUCUUCCUCGG
UACAUAAUCUCCUUUGGCGUUUCCCGAUGUCCGUCACGCACAUGGGAUC
CCCGGGUACCGAG
SEQ ID No: 3
   **PP1 oligonucleotide**: 
5'-
GTATCGCGAAAATGTAAAGCAATGCTGCTGCTGTACTACGAGCGGTCTCC
AAGGAATGCGCATTTAGGAAGAATGTCGCTGGACG
SEQ ID No: 4
   **PP2 oligonucleotide:** 
5’-
ATGTCGCTGGACGGTAATGCTGCTGCTGTACTACGAGCGGTCTCCAGGAA
TGCGCATTTATGTACCGAGGAAGA
SEQ ID No: 5
   **PP3 oligonucleotide:** 
5'-
TATGTACCGAGGAAGATGATGCTGCTGCTGTACTACGAGCGGTCTCCAGG
AACGCATGAAACGCCAAAGGAGAT
SEQ ID No: 6
   **PP4 oligonucleotide:** 
5'-.
GTTTTACAACGCCAAGCTTGCATGAATGCAGGTTAGGATCCAATGGTACC
GAGCTCGAATTCACTGGCCGTC
SEQ ID No: 7
   **PP5 oligonucleotide:** 
5'-
TGAGCACAGGGTACTATGCTGCTGCTGTACTACGAGCGGTCTCCAGGAAT
GCGCATTTTTTTTTTTTGGT
SEQ ID No: 8
   **PP6 oligonucleotide:** 
5’-
AGAGCACAGGGTACTATGCTGCTGCTGTACTACGAGCGGTCTCCAGGAAT
GCGCATTTTTTTTTTTTGGA
SEQ ID No: 9
   **PP7 oligonucleotide:** 
5’-
TTTATTCAACTGGTCATGCTGCTGCTGTACTACGAGCGGTCTCCAGGAATG
CGCATTTAAGGTGTGCACT
SEQ ID No: 10
   **PP8 oligonucleotide:** 
5’-
ATTATTCAACTGGTCATGCTGCTGCTGTACTACGAGCGGTCTCCAGGAATG
CGCATTTAAGGTGTGCACA
SEQ ID No: 11
   **PP9 oligonucleotide** 5’- 
GCTAAGCAGTTGGTGATGCTGCTGCTGTACTACGAGCGGTCTCCAGGAAT
GCGCAACCTTGGCCAGGGGG
SEQ ID No: 12
   **PP10 oligonucleotide** 
5’-
GCTAAGCAGTTGGTGATGCTGCTGCTGTACTACGAGCGGTCTCCAGGAAT
GCGCAACCTTGGCCAGGGGT
SEQ ID No: 13
   **PP11 oligonucleotide:** 
5’-
CTGCCCAGGGCCTCAATGCTGCTGCTGTACTACGAGCGGTCTCCAGGAAT
GCGCAGTAGACAACCAGCGC
SEQ ID No: 14
   **PP12 oligonucleotide:** 
5'-
TCAACTGGTCTCAAGATGCTGCTGCTGTACTACGAGCGGTCTCCAGGAAT
GCGCAGGTGTGCACTTTTAT'
SEQ ID No: 15
   **PP13 oligonucleotide:** 
5'-
TGGTCTCAAGTCAGTATGCTGCTGCTGTACTACGAGCGGTCTCCAGGAATG
CGCAGGTGTGCACTTTTAT
SEQ ID No: 16
   **PL1 oligonucleotide:**
      5'-CGTTTACATTTTCGCGATACCGTCCAGCGACATTCTTCCT
SEQ ID No: 17
   **PL2 oligonucleotide**
      5'- ACCGTCCAGCGACATTCTTCCTCGGTACAT
SEQ ID No: 18
   **PL3 oligonucleotide:**
      5'-CTTCCTCGGTACATAATCTCCTTTGGCGTT
SEQ ID No: 19
   **PL4 oligonucleotide:**
      5'- TTGGCGTTGTAAAACGACGGCCAGTGAATT
SEQ ID No: 20
   **PL5 oligonucleotide:**
      5'-AGTACCCTGTGCTCAACCAAAAAAAAAAAA
SEQ ID No: 21
   **PL6 oligonucleotide:**
      5'-AGTACCCTGTGCTCTTCCAAAAAAAAAAAA
SEQ ID No: 22
   **DP-FITC oligonucleotide:**
      5'-Fluorescein-AATGCTGCTGCTGTACTACGG
SEQ ID No: 23
   **Oligonucleotide (for RCA product cleavage with Mva1269I restriction enzyme):**
      5'-CCAGGAATGCGCATTTATGT
SEQ ID No: 24
   **Oligonucleotide (for RCA product cleavage with RsaI restriction enzyme):**
      5'-CCTCAATGCTGCTGCTGTACTACGAGG
SEQ ID No: 25
   **Bacteriophage T4 DNA polymerase amino acid sequence (UniProt P04415)** 
MKEFYISIET VGNNIVERYI DENGKERTRE VEYLPTMFRH
CKEESKYKDI YGKNCAPQKF PSMKDARDWM KRMEDIGLEA
LGMNDFKLAY ISDTYGSEIV YDRKFVRVAN CDIEVTGDKF
PDPMKAEYEI DAITHYDSID DRFYVFDLLN SMYGSVSKWD
AKLAAKLDCE GGDEVPQEIL DRVIYMPFDN ERDMLMEYIN
LWEQKRPAIF TGWNIEGFDV PYIMNRVKMI LGERSMKRFS
PIGRVKSKLI QNMYGSKEIY SIDGVSILDY LDLYKKFAFT
NLPSFSLESV AQHETKKGKL PYDGPINKLR ETNHQRYISY
NIIDVESVQA IDKIRGFIDL VLSMSYYAKM PFSGVMSPIK
TWDAIIFNSL KGEHKVIPQQ GSHVKQSFPG AFVFEPKPIA
RRYIMSFDLT SLYPSIIRQV NISPETIRGQ FKVHPIHEYI
AGTAPKPSDE YSCSPNGWMY DKHQEGIIPK EIAKVFFQRK
DWKKKMFAEE MNAEAIKKII MKGAGSCSTK PEVERYVKFS
DDFLNELSNY TESVLNSLIE ECEKAATLAN TNQLNRKILI
NSLYGALGNI HFRYYDLRNA TAITIFGQVG IQWIARKINE
YLNKVCGTND EDFIAAGDTD SVYVCVDKVI EKVGLDRFKE
QNDLVEFMNQ FGKKKMEPMI DVAYRELCDY MNNREHLMHM
DREAISCPPL GSKGVGGFWK AKKRYALNVY DMEDKRFAEP
HLKIMGMETQ QSSTPKAVQE ALEESIRRIL QEGEESVQEY
YKNFEKEYRQ LDYKVIAEVK TANDIAKYDD KGWPGFKCPF
HIRGVLTYRR AVSGLGVAPI LDGNKVMVLP LREGNPFGDK
CIAWPSGTEL PKEIRSDVLS WIDHSTLFQK SFVKPLAGMC
ESAGMDYEEK ASLDFLFG
SEQ ID No. 26
   **Bacteriophage T7 DNA polymerase amino acid sequence (UniProt P00581)** 
MIVSDIEANA LLESVTKFHC GVIYDYSTAE YVSYRPSDFG
AYLDALEAEV ARGGLIVFHN GHKYDVPALT KLAKLQLNRE
FHLPRENCID TLVLSRLIHS NLKDTDMGLL RSGKLPGKRF
 
GSHALEAWGY RLGEMKGEYK DDFKRMLEEQ GEEYVDGMEW
WNFNEEMMDY NVQDVVVTKA LLEKLLSDKH YFPPEIDFTD
VGYTTFWSES LEAVDIEHRA AWLLAKQERN GFPFDTKAIE
ELYVELAARR SELLRKLTET FGSWYQPKGG TEMFCHPRTG
KPLPKYPRIK TPKVGGIFKK PKNKAQREGR EPCELDTREY
VAGAPYTPVE HVVFNPSSRD HIQKKLQEAG WVPTKYTDKG
APVVDDEVLE GVRVDDPEKQ AAIDLIKEYL MIQKRIGQSA
EGDKAWLRYV AEDGKIHGSV NPNGAVTGRA THAFPNLAQI
PGVRSPYGEQ CRAAFGAEHH LDGITGKPWV QAGIDASGLE
LRCLAHFMAR FDNGEYAHEI LNGDIHTKNQ IAAELPTRDN
AKTFIYGFLY GAGDEKIGQI VGAGKERGKE LKKKFLENTP
AIAALRESIQ QTLVESSQWV AGEQQVKWKR RWIKGLDGRK
VHVRSPHAAL NTLLQSAGAL ICKLWIIKTE EMLVEKGLKH
GWDGDFAYMA WVHDEIQVGC RTEEIAQVVI ETAQEAMRWV
GDHWNFRCLL DTEGKMGPNW AICH
SEQ ID No. 27
   **Bacteriophage phi-29 DNA polymerase amino acid sequence (UniProt Q38545)**

MKHMPRKMYS CDFETTTKVE DCRVWAYGYM NIEDHSEYKI
GNSLDEFMAW VLKVQADLYF HNLKFDGAFI INWLERNGFK
WSADGLPNTY NTIISRMGQW YMIDICLGYK GKRKIHTVIY
DSLKKLPFPV KKIAKDFKLT VLKGDIDYHK ERPVGYKITP
EEYAYIKNDI QIIAERLLIQ FKQGLDRMTA GSDSLKGFKD
IITTKKFKKV FPTLSLGLDK EVRYAYRGGF TWLNDRFKEK
EIGEGMVFDV NSLYPAQMYS RLLPYGEPIV FEGKYVWDED
YPLHIQHIRC EFELKEGYIP TIQIKRSRFY KGNEYLKSSG
GEIADLWLSN VDLELMKEHY DLYNVEYISG LKFKATTGLF
KDFIDKWTYI KTTSEGAIKQ LAKLMLNSLY GKFASNPDVT
GKVPYLKENG ALGFRLGEEE TKDPVYTPMG VFITAWARYT
TITAAQACYD RIIYCDTDSI HLTGTEIPDV IKDIVDPKKL

GYWAHESTFK RVKYLRQKTY IQDIYMKEVD GKLVEGSPDD
YTDIKFSVKC AGMTDKIKKE VTFENFKVGF SRKMKPKPVQ
VPGGVVLVDD TFTIK

### REFERENCES

Baner *et al.:* Signal amplification of padlock probes by rolling circle replication. *Nucleic Acids Research,* 1998, Vol 26, No.22, 5073-5078.
Baner *et al.:* More keys to padlock probes:mechanisms for high-throughput nucleic acid analysis. *Current Opinion Biotechnology,* 2001, Vol. 12, No1, 11-15.
Baner *et al.:* Parallel gene analysis with allele-specific padlock probes and tag microarrays. *Nucleic Acids Research,* 2003, Vol. 31, No. 17, 1-7.
Baner *et al.:* Analysis of T-Cell Receptor Vβ Gene Repertoires after Immune Stimulation and in Malignancy by Use of Padlock Probes and Microarrays. *Clinical Chemistry,* 2005, Vol.51:4: 768-775.
Christian *et al.:* Detection of DNA point mutations and mRNA expression levels by rolling circle amplification in individual cells. *PNAS,* 2001, Vol.98, No.25, 14238-14243.
Gorshkova *et al.:* HIV-1 reverse transcriptase interaction with model RNA-DNA duplexes. *Anal. Biochem.,* 2001, Vol. 291, No. 2, 198-206
Haible *et al.:* Rolling circle amplification revolutionizes diagnosis and genomics of geminiviruses. *Journal of Virological Methods,* 2006, Vo1.135, No.1, 9-16.
Jonstrup et al.: A microRNA detection system based on padlock probes and rolling circle amplication. RNA, 2006, Vo1.12, 1747-1752.
Kirkpatrick *et al.:* RNA-DNA hybridization promoted by *E. coli* RecA protein. *Nucleic Acids Research,* Vol.20, No.16, 4339-4346
Landegren *et al.:* Molecular tools for a molecular medicine: analyzing genes, transcripts and proteins using padlock and proximity probes. *Journal of Molecular Recognition,* 2004, Vol. 17, 194-197
Larsson *et al.: In situ* genotyping individual DNA molecules by target-primed rolling-circle amplification of padlock probes. *Nature Methods,* 2004, Vol.1, No.3, 227-232
Lizardi *et al.:* Mutation detection and single-molecule counting using isothermal rolling-circle amplification. *Nature Genetics,* 1998, Vol. 19, 225-232
Lowman *et al.:* On the Recognition of Helical RNA by Cobra Venom V, Nuclease. *The Journal of Biological Chemistry,* 1986, Vol.26 1, No.12, 5396-5406
March *et al.:* Characterization of the biochemical properties of recombinant ribonuclease III. *Nucleic Acids Research,* Vo1.18, No.11, 3293
Nakai and Richardson: Dissection of RNA-primed DNA synthesis catalyzed by gene 4 protein and DNA polymerase of bacteriophate T7. *J Biol. Chem.,* 1986, Vol. 261, No. 32, 15217-15224. Niel *et al.:* Rolling-circle amplification of *Torque teno virus* (TTV) complete genomes for human and swine sera and indication of novel swine TTV genogroup. *Journal of Gener-al Virology,* 2005, Vol.86, 1343-1347
Nilsson *et al.:* RNA-templated DNA ligation for transcript analysis. *Nucleic Acids Research,* 2001, Vol.29, No.2, 578-581 1
Powell *et al.:* Residues in the αH and αI helices of the HIV-1 reverse transcriptase thumb subdomain are required for the specificity of RNase H-catalyzed removal of the polypurine tract primer. *J*. *Biol. Chem.,* 1999, Vol. 274, No. 28, 19885-19893.
Ramadan *et al.:* Human DNA polymerases, possesses terminal deoxyribonucleotidyl transferase activity and can elongate RNA primers, *J*. *Mol. Biol.,* 2003, Vol 328, No. 1, 63-72.
Zhou *et al.: In Situ* Detection of Messenger RNA Using Digoxigenin-Labeled Oligonucleotides and Rolling Circle Amplification. *Experimental and Molecular Pathology,* 2001, Vol.70, 281-288.

## Claims

1. Use of a DNA polymerase enzyme as a single stranded RNA exoribonuclease.

2. Use according to claim 1 wherein the DNA polymerase has a 3'-5' exonuclease sequence or 3'-5' exonuclease domain, which sequence or domain has a fold containing a ribonuclease H-like structure motif.

3. Use according to claim 1 or claim 2, wherein the DNA polymerase is T7 DNA polymerase, T4 DNA polymerase or Phi29 DNA polymerase.

4. Use according to any preceding claim in a method for polynucleotide production.

5. Use according to claim 4 wherein the polynucleotide production is DNA synthesis or amplification of DNA.

6. Use according to any preceding claim in a method for *in situ* detection of RNA.

7. Use according to claim 6 wherein the method is for *in situ* detection of an RNA loci or species, a single nucleotide polymorphism (SNP), a splice variant, or a deletion or insertion in the RNA.

8. Use of a DNA polymerase enzyme according to claim 1 as a combined polymerase and single stranded RNA exoribonuclease.

9. Use of a DNA polymerase enzyme according to claim 1 for conversion of an RNA target into a primer for DNA polymerisation.

10. Use of a DNA polymerase enzyme according to claim 1 for production of a ribonucleotide.

11. Use of a DNA polymerase enzyme according to claim 1 for producing a RNA-DNA fusion molecule.

12. Use according to any one of claims 8 to 11 wherein the DNA polymerase has a 3'-5' exonuclease sequence or 3'-5' exonuclease domain, which sequence or domain has a fold containing a ribonuclease H-like structure motif.

13. Use according to any one of claims 8 to 12 wherein the DNA polymerase is T7 DNA polymerase, T4 DNA polymerase or Phi29 DNA polymerase.

14. A process for hydrolysing a single stranded RNA sequence comprising contacting the RNA sequence with a DNA polymerase enzyme having exoribonuclease activity.

15. A process for polynucleotide production from a target region within a single stranded RNA sequence comprising:
a) forming a padlock probe-target region hybrid;
b) adding a DNA polymerase enzyme capable of acting as a single stranded RNA exoribonuclease;
c) allowing the enzyme to hydrolyse the single stranded RNA sequence; and
d) allowing the enzyme to act as a polymerase to produce the polynucleotide in the presence of dNTPs,
wherein the process is conducted in the absence of an exogenous primer.

16. A process according to claim 14 or claim 15 wherein the enzyme is contacted with the RNA sequence in the presence of a divalent metal ion.

17. A process according to claim 16 wherein the divalent metal ion is selected from Mg2+, Mn2+ and Co2+.

18. A process according to any one of claims 14 to 17 wherein the polymerase is contacted with the RNA molecule in the present of RNAse.

19. A process according to claim 18 wherein the RNAse is selected from RNAse III or RNAse VI.

20. A process according to any one of claims 15 to 19 wherein step a) is performed in the presence of Rec A protein.

21. A process according to any one of claims 15 to 20 for detecting an RNA molecule comprising the RNA sequence wherein a labelled probe is contacted with the produced polynucleotide to detect the RNA molecule.

22. A process according to claim 21 wherein the RNA molecule is detected *in situ.*

23. A process according to claim 21 wherein the RNA molecule is detected *in vitro.*

24. A process according to claim 23 wherein the RNA molecule is detected *in vitro* from total mRNA.

25. A process according to any one of claims 15 to 20 for exponential amplification of polynucleotide from the target region within the RNA sequence further comprising the steps of:
e) adding a DNA primer complementary to the produced polynucleotide, and
f) allowing the enzyme to act as a polymerase to produce further copies of polynucleotide in the presence of dNTPs from the produced polynucleotide.

26. A process according to any one of claims 14 to 25 wherein the DNA polymerase has a 3'-5' exonuclease sequence or 3'-5' exonuclease domain, which sequence or domain has a fold containing a ribonuclease H-like structure motif.

27. A process according to any one of claims 14 to 26 wherein the DNA polymerase is T7 DNA polymerase, T4 DNA polymerase or Phi29 DNA polymerase.

28. A process according to any one of claims 14 to 27 wherein the RNA sequence is an mRNA.

29. A kit for polynucleotide production from a target region within a single stranded RNA sequence comprising:
a) a padlock probe or a padlock probe precursor
b) a DNA polymerase capable of acting as a single stranded RNA exoribonuclease
wherein the kit does not contain a further primer other than the probe or probe precursor.

30. A kit according to claim 29 further comprising dNTPs.

31. A kit according to claim 29 or claim 30 further comprising a DNA ligase

32. A kit according to any one of claims 29 to 31 further comprising instructions for using the polymerase as an exoribonuclease.

33. A kit according to any one of claims 29 to 32 further comprising instructions for using the kit in the absence of an exogenous primer.

34. A kit according to any one of claims 29 to 33 for detection of RNA targets *in situ.*

35. A kit according to claim 34 for detection *in situ* of an RNA locus or species, a single nucleotide polymorphism (SNP), a splice variant, or a deletion or insertion in the RNA.

36. A kit according to any one of claims 29 to 35 further comprising a labelled hybridisation probe.

37. A kit according to any one of claims 29 to 36, further comprising an RNase.

38. A kit according to any one of claims 29 to 37, further comprising a RecA protein.

39. A kit according to any one of claims 29 to 38 wherein the DNA polymerase has a 3'-5' exonuclease sequence or 3'-5' exonuclease domain, which sequence or domain has a fold containing a ribonuclease H-like structure motif.

40. A kit according to any one of claims 29 to 39 wherein the DNA polymerase is T7 DNA polymerase, T4 DNA polymerase or Phi29 DNA polymerase.

41. Use of a kit according to any one of claims 29 to 40 for polynucleotide production.

## Patentansprüche

1. Verwendung eines DNA-Polymerase-Enzyms als eine einsträngige RNA-Exoribonuclease.

2. Verwendung nach Anspruch 1, wobei die DNA-Polymerase eine 3'-5'-Exonuclease-Sequenz oder 3'-5'-Exonuclease-Domäne aufweist, wobei die Sequenz bzw.Domäne eine Faltung hat, welche ein Ribonuklease-H-artiges Strukturmotiv enthält.

3. Verwendung nach Anspruch 1 oder 2, wobei die DNA-Polymerase T7DNA-Polymerase, T4DNA-Polymerase oder Phi29 DNA-Polymerase ist.

4. Verwendung nach einem der vorhergehenden Ansprüche in einem Verfahren zur Polynucleotid-Produktion.

5. Verwendung nach Anspruch 4, wobei die Polynucleotid-Produktion DNA-Synthese oder Amplifikation von DNA ist.

6. Verwendung nach einem der vorhergehenden Ansprüche in einem Verfahren zur *in situ* Detektion von RNA.

7. Verfahren nach Anspruch 6, wobei das Verfahren zur in situ Detektion von RNA-Loci oder -Species, eines Einzelnukleotid-Polymorphismus (SNP), einer Splicevariante, oder einer Deletion oder Insertion in der RNA ist.

8. Verwendung eines DNA-Polymerase-Enzyms nach Anspruch 1 als eine kombinierte Polymerase und einzelsträngige RNA-Exoribonuclease.

9. Verwendung eines DNA-Polymerase-Enzyms nach Anspruch 1 zur Konversion eines RNA-Targets in einen Primer zur DNA-Polymerisation.

10. Verwendung eines DNA-Polymerase-Enzyms nach Anspruch 1 zur Produktion eines Ribonucleotids.

11. Verwendung eines DNA-Polymerase-Enzyms nach Anspruch 1 zur Produktion eines RNA-DNA-Fusions-Moleküls.

12. Verwendung nach einem der Ansprüche 8 bis 11, wobei die DNA-Polymerase eine 3'-5'-Exonuclease-Sequenz oder 3'-5'-Exonuclease-Domäne hat, welche Sequenz oder Domäne eine Faltung hat, welche ein Ribonuclease-H-artiges Strukturmotiv enthält.

13. Verwendung nach einem der Ansprüche 8 bis 12, wobei die DNA-Polymerase T7 DNA-Polymerase, T4 DNA-Polymerase oder Phi29-DNA-Polymerase ist.

14. Verfahren zum Hydrolysieren einer einsträngigen RNA-Sequenz umfassend das Kontaktieren der RNA-Sequenz mit einem DNA-Polymerase-Enzym, welches Exoribonuclease-Aktivität aufweist.

15. Verfahren zur Polynucleotid-Produktion aus einer Target-Region in einer einsträngigen RNA-Sequenz, umfassend:
a) Bilden einer Padlock-Sonden-Target-Region hybrid;
b) Hinzufügen eines DNA-Polymerase-Enzyms, welches in der Lage ist, als eine einsträngige RNA-Exoribonuclease zu agieren;
c) dem Enzym erlauben, die einsträngige RNA-Sequenz zu hydrolysieren; und
d) dem Enzym erlauben, als eine Polymerase zu agieren, um das Polynucleotid in der Gegenwart von dNTPs zu produzieren,
wobei das Verfahren in der Abwesenheit eines exogenen Primers durchgeführt wird.

16. Verfahren nach Anspruch 14 oder 15, wobei das Enzym in der Gegenwart eines divalenten Metallions mit der RNA-Sequenz kontaktiert wird.

17. Verfahren nach Anspruch 16, wobei das divalenteMetallion aus Mg2+, Mn2+ und Co2+ ausgewählt ist.

18. Verfahren nach einem der Ansprüche 14 bis 17, wobei die Polymerase mit dem RNA-Molekül in der Gegenwart von RNAse kontaktiert wird.

19. Verfahren nach Anspruch 18, wobei die RNAse aus RNAse III oder RNAse VI ausgewählt ist.

20. Verfahren nach einem der Ansprüche 15 bis 19, wobei Schritt a) in der Gegenwart von RecA Protein durchgeführt wird.

21. Verfahren nach einem der Ansprüche 15 bis 20 zur Detektion eines RNA-Moleküls umfassend die RNA-Sequenz, wobei eine gelabelte Sonde mit dem produzierten Polynucleotid kontaktiert wird, um das RNA-Molekül zu detektieren.

22. Verfahren nach Anspruch 21, wobei das RNA-Molekül *in si*tu detektiert wird.

23. Verfahren nach Anspruch 21, wobei das RNA-Molekül in vitro detektiert wird.

24. Verfahren nach Anspruch 23, wobei das RNA-Molekül in vitro von Total-mRNA detektiert wird.

25. Verfahren nach einem der Ansprüche 15 bis 20 zur exponentiellen Amplifikation von Polynucleotid aus der Target-Region in der RNA-Sequenz, ferner umfassend die Schritte:
e) Hinzufügen eines DNA-Primers komplementär zu dem produzierten Polynucleotid, und
f) dem Enzym erlauben, als eine Polymerase zu agieren, um weitere Kopien von Polynucleotid in der Gegenwart von dNTPs aus dem produzierten Polynucleotid zu produzieren.

26. Verfahren nach einem der Ansprüche 14 bis 25, wobei die DNA-Polymerase eine 3'-5'-Exonuclease-Sequenz oder eine 3'-5'-Exonuclease-Domäne hat, welche Sequenz oder Domäne eine Faltung hat, welche ein Ribonuclease-H-artiges Strukturmotiv enthält.

27. Verfahren nach einem der Ansprüche 14 bis 26, wobei die DNA-Polymerase T7 DNA-Polymerase, T4 DNA-Polymerase oder Phi29 DNA-Polymerase ist.

28. Verfahren nach einem der Ansprüche 14 bis 27, wobei die RNA-Sequenz eine mRNA ist.

29. Kit für Polynucleotid-Produktion aus einer Target-Region in einer einsträngigen RNA-Sequenz, umfassend:
a) eine Padlock-Sonde oder einen Padlock-Sonden-Präkursor
b) eine DNA-Polymerase, welche in der Lage ist, als eine einsträngige RNA-Exoribonuclease zu agieren, wobei der Kit keinen weiteren Primer als die Sonde oder den Sonden-Präkursor enthält.

30. Kit nach Anspruch 29, ferner aufweisend dNTPs.

31. Kit nach Anspruch 29 oder 30, ferner aufweisend eine DNA-Ligase.

32. Kit nach einem der Ansprüche 29 bis 31, ferner aufweisend Instruktionen zur Verwendung der Polymerase als eine Exoribonuclease.

33. Kit nach einem der Ansprüche 29 bis 32, ferner aufweisend Instruktionen zur Verwendung des Kits in der Abwesenheit eines exogenen Primers.

34. Kit nach einem der Ansprüche 29 bis 33 zur Detektion von RNA-Targets *in situ.*

35. Kit nach Anspruch 34 zur Detektion in situeines RNA-Locus oder -Species, eines Einzelnucleotid-Polymorphismus (SNP), einer Splicevariante, oder einer Deletion oder Insertion in der RNA.

36. Kit nach einem der Ansprüche 29 bis 35, ferner umfassend eine gelabelte Hybridisierungssonde.

37. Kit nach einem der Ansprüche 29 bis 36, ferner umfassend eine RNase.

38. Kit nach einem der Ansprüche 29 bis 37, ferner umfassend ein RecAProtein.

39. Kit nach einem der Ansprüche 29 bis 38, wobei die DNA-Polymerase eine 3'-5'-Exonuclease-Sequenz oder 3'-5'-Exonuclease-Domäne hat, welche Sequenz oder Domäne eine Faltung hat, welche ein Ribonuclease-H-artiges-Strukturmotiv enthält.

40. Kit nach einem der Ansprüche 29 bis 39, wobei die DNA-Polymerase T7 DNA-Polymerase, T4 DNA-Polymerase oder Phi29 DNA-Polymeraseist.

41. Verwendung eines Kits nach einem der Ansprüche 29 bis 40 zur Polynucleotid-Produktion.

## Revendications

1. Utilisation d'une enzyme ADN-polymérase comme exoribonucléase d'ARN monocaténaire.

2. Utilisation suivant la revendication 1, dans laquelle l'ADN-polymérase a une séquence d'exonucléase 3'-5' ou un domaine d'exonucléase 3'-5', séquence ou domaine qui comporte un pli contenant un motif structural analogue à la ribonucléase H.

3. Utilisation suivant la revendication 1 ou la revendication 2, dans laquelle l'ADN-polymérase est l'ADN-polymérase T7, l'ADN-polymérase T4 ou l'ADN-polymérase Phi29.

4. Utilisation suivant l'une quelconque des revendications précédentes dans un procédé pour la production de polynucléotides.

5. Utilisation suivant la revendication 4, dans laquelle la production de polynucléotides est une synthèse d'ADN ou une amplification d'ADN.

6. Utilisation suivant l'une quelconque des revendications précédentes dans un procédé pour la détection in situ d'ARN.

7. Utilisation suivant la revendication 6, dans laquelle le procédé est un procédé pour la détection in situ d'un locus ou type d'ARN, d'un polymorphisme de nucléotide unique (SNP), d'un variant d'épissage ou d'une délétion ou insertion dans l'ARN.

8. Utilisation d'une enzyme ADN-polymérase suivant la revendication 1 sous forme d'une polymérase et d'une exoribonucléase d'ARN monocaténaire combinées.

9. Utilisation d'une enzyme ADN-polymérase suivant la revendication 1 pour la conversion d'une cible ARN en une amorce pour la polymérisation de l'ADN.

10. Utilisation d'une enzyme ADN-polymérase suivant la revendication 1 pour la production d'un ribonucléotide.

11. Utilisation d'une enzyme ADN-polymérase suivant la revendication 1 pour la production d'une molécule de fusion ARN-ADN.

12. Utilisation suivant l'une quelconque des revendications 8 à 11, dans laquelle l'ADN-polymérase a une séquence d'exonucléase 3'-5' ou un domaine d'exonucléase 3'-5', séquence ou domaine qui comporte un pli contenant un motif structural analogue à la ribonucléase H.

13. Utilisation suivant l'une quelconque des revendications 8 à 12, dans laquelle l'ADN-polymérase est l'ADN-polymérase T7, l'ADN-polymérase T4 ou l'ADN-polymérase Phi29.

14. Procédé pour l'hydrolyse d'une séquence d'ARN monocaténaire, comprenant la mise en contact de la séquence d'ARN avec une enzyme ADN-polymérase ayant une activité d'exoribonucléase.

15. Procédé pour la production d'un polynucléotide à partir d'une région cible dans une séquence d'ARN monocaténaire, comprenant les étapes consistant .
a) à former un hybride sonde en cadenas-région cible ;
b) à ajouter une enzyme ADN-polymérase capable de jouer le rôle d'exoribonucléase d'ARN monocaténaire ;
c) à laisser l'enzyme hydrolyser la séquence d'ARN monocaténaire ; et
d) à laisser l'enzyme jouer le rôle de polymérase pour produire le polynucléotide en présence des dNTP,
ledit procédé étant mis en oeuvre en l'absence d'amorce exogène.

16. Procédé suivant la revendication 14 ou la revendication 15, dans lequel l'enzyme est mise en contact avec la séquence d'ARN en présence d'un ion métallique divalent.

17. Procédé suivant la revendication 16, dans lequel l'ion métallique divalent est choisi entre Mg²⁺, Mn²⁺ et Co²⁺.

18. Procédé suivant l'une quelconque des revendications 14 à 17, dans lequel la polymérase est mise en contact avec la molécule d'ARN en présence de RNAse.

19. Procédé suivant la revendication 18, dans lequel la RNAse est choisie entre la RNAse III et la RNAse VI.

20. Procédé suivant l'une quelconque des revendications 15 à 19, dans lequel l'étape a) est mise en ouvre en présence de la protéine Rec A.

21. Procédé suivant l'une quelconque des revendications 15 à 20 pour la détection d'une molécule d'ARN comprenant la séquence d'ARN, dans lequel une sonde marquée est mise en contact avec le polynucléotide produit pour détecter la molécule d'ARN.

22. Procédé suivant la revendication 21, dans lequel la molécule d'ARN est détectée in situ.

23. Procédé suivant la revendication 21, dans lequel la molécule d'ARN est détectée in vitro.

24. Procédé suivant la revendication 23, dans lequel la molécule d'ARN est détectée in vitro à partir de l'ARNm total.

25. Procédé suivant l'une quelconque des revendications 15 à 20 pour l'amplification exponentielle d'un polynucléotide à partir de la région cible dans la séquence d'ARN, comprenant en outre les étapes consistant :
e) à ajouter une amorce d'ADN complémentaire du polynucléotide produit, et
f) à laisser l'enzyme jouer le rôle de polymérase pour produire des copies supplémentaires du polynucléotide en présence des DNTP à partir du nucléotide produit.

26. Procédé suivant l'une quelconque des revendications 14 à 25, dans lequel l'ADN-polymérase a une séquence d'exonucléase 3'-5' ou un domaine d'exonucléase 3'-5', séquence ou domaine qui comporte un pli contenant un motif structural analogue à la ribonucléase H.

27. Procédé suivant l'une quelconque des revendications 14 à 26, dans lequel l'ADN-polymérase est l'ADN-polymérase T7, l'ADN-polymérase T4 ou l'ADN-polymérase Phi29.

28. Procédé suivant l'une quelconque des revendications 14 à 27, dans lequel la séquence d'ARN est un ARNm.

29. Kit pour la production d'un polynucléotide à partir d'une région cible dans une séquence d'ARN monocaténaire, comprenant :
a) une sonde en cadenas ou un précurseur de sonde en cadenas
b) une ADN-polymérase capable de jouer le rôle d'exoribonucléase d'ARN monocaténaire,
ledit kit ne contenant pas d'amorce supplémentaire autre que la sonde ou le précurseur de sonde.

30. Kit suivant la revendication 29, comprenant en outre des dNTP.

31. Kit suivant la revendication 29 ou la revendication 30, comprenant en outre une ADN-ligase.

32. Kit suivant l'une quelconque des revendications 29 à 31, comprenant en outre des instructions pour l'utilisation de la polymérase comme exoribonucléase.

33. Kit suivant l'une quelconque des revendications 29 à 32, comprenant en outre des instructions pour l'utilisation du kit en l'absence d'amorce exogène.

34. Kit suivant l'une quelconque des revendications 29 à 33 pour la détection de cibles ARN in situ.

35. Kit suivant la revendication 34 pour la détection in situ d'un locus ou type d'ARN, d'un polymorphisme de nucléotide unique (SNP), d'un variant d'épissage ou d'une délétion ou insertion dans l'ARN.

36. Kit suivant l'une quelconque des revendications 29 à 35, comprenant en outre une sonde d'hybridation marquée.

37. Kit suivant l'une quelconque des revendications 29 à 36, comprenant en outre une RNase.

38. Kit suivant l'une quelconque des revendications 29 à 37, comprenant en outre une protéine RecA.

39. Kit suivant l'une quelconque des revendications 29 à 38, dans lequel l'ADN-polymérase a une séquence d'exonucléase 3'-5' ou un domaine d'exonucléase 3'-5', séquence ou domaine qui comporte un pli contenant un motif structural du type ribonucléase H.

40. Kit suivant l'une quelconque des revendications 29 à 39, dans lequel l'ADN-polymérase est l'ADN-polymérase T7, l'ADN-polymérase T4 ou l'ADN-polymérase Phi29.

41. Utilisation d'un kit suivant l'une quelconque des revendications 29 à 40 pour la production de polynucléotides.
